# EUROPÄISCHE PATENTANMELDUNG

(11) **EP 2 241 557 A1**
(43) Veröffentlichungstag der Anmeldung: **20.10.2010**
(21) Anmeldenummer: 09157141.4
(22) Anmeldetag: 02.04.2009
(51) Int. Cl.: C07D 241/44, C07D 401/12, C07D 409/12, C07D 413/12, A61K 31/498, A61P 35/00

(54) **Chinoxalin-Derivate und deren Anwendung zur Behandlung gutartiger und bösartiger Tumorerkrankungen**

(71) Anmelder: Æterna Zentaris GmbH, 60314 Frankfurt am Main (DE)
(72) Erfinder: Gerlach, Matthias, 63636, Brachttal (DE); Seipelt, Irene, 63069 Offenbach (DE); Günther, Eckhard, 63477 Maintal (DE); Schuster, Tilmann, 63762 Grossostheim (DE); Polymeropoulos, Emmanuel, 60325 Frankfurt am Main (DE); Czech, Michael, 60435 Frankfurt am Main (DE); Claus, eckhard, 60388 Frankfurt am Main (DE)

(57) **Zusammenfassung**

Gegenstand der Erfindung sind Chinoxaline der allgemeinen Formel I, die als Arzneimittel bevorzugt zur Behandlung von Tumorerkrankungen, insbesondere bei Arzneimittelresistenz gegen andere Wirkstoffe und bei metastasierendem Karzinom eingesetzt werden.

Die möglichen Anwendungen sind nicht auf Tumorerkrankungen beschränkt.

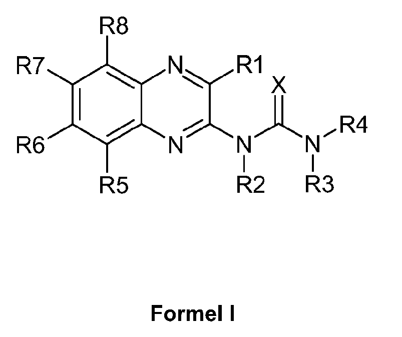

## Beschreibung

### Technisches Gebiet

Die vorliegende Erfindung betrifft Chinoxalin-Derivate, deren Herstellung sowie deren Verwendung als Arzneimittel, insbesondere zur Behandlung von gutartigen und bösartigen Tumoren am Menschen und Säugetier.

### Stand der Technik

Für die nächsten Jahre wird ein dramatischer Anstieg der Tumorerkrankungen und der Tumor bedingten Todesfälle weltweit erwartet. In 2001 waren weltweit etwa 10 Mio Menschen an Krebs erkrankt und über 6 Mio Menschen sind an dieser Erkrankung gestorben. Die Entwicklung von Tumoren ist eine fundamentale Erkrankung höherer Organismen im Pflanzen-, im Tierreich und beim Menschen. Das allgemein anerkannte Mehrschrittmodell der Krebsentstehung geht davon aus, daß durch Anhäufung von mehreren Mutationen in einer einzelnen Zelle diese so in ihrem Proliferations- und Differenzierungsverhalten geändert wird, daß letztendlich über benigne Zwischenstufen ein maligner Zustand mit Metastasierung erreicht wird. Hinter dem Begriff Krebs oder Tumor verbirgt sich ein Krankheitsbild mit mehr als 200 verschiedenen Einzelerkrankungen. Tumorerkrankungen können gutartig oder bösartig verlaufen. Die wichtigsten Tumoren sind die der Lunge, der Brust, des Magens, des Gebärmutterhalses, der Prostata, des Kopfes und Halses, des Dick- und Enddarmes, der Leber und des Blutsystems. Hinsichtlich Verlauf, Prognose und Therapieverhalten gibt es große Unterschiede. Mehr als 90% der erkannten Fälle betreffen solide Tumoren, die insbesondere in fortgeschrittenem Stadium bzw. bei Metastasierung schwer oder nicht therapierbar sind. Die drei Säulen der Krebsbekämpfung sind nach wie vor operative Entfernung, Bestrahlung und Chemotherapie. Trotz großer Fortschritte ist es bisher nicht gelungen, Medikamente zu entwickeln, die bei den weitverbreiteten soliden Tumoren eine deutliche Verlängerung der Überlebenszeit oder gar komplette Heilung bewirken. Es ist deshalb sinnvoll, neue Arzneimittel zur Bekämpfung der Krebserkrankung zu erfinden.

Chinoxalinderivate finden als pharmakodynamisch aktive Verbindungen und als Synthesebausteine in der pharmazeutischen Chemie eine vielfältige Verwendung.

In dem Dokument WO08/141065 A1 werden Chinoxalin-Derivate als PI3K-Inhibitoren, in der Patentschrift US2007/0254894 als Angiogenese-Inhibitoren, in der Patentschrift WO08/015423 als CHK-Inhibitoren, in den Patenschriften WO9854156, WO9854157, WO9854158, WO2000031049, WO2000031050 und WO2000031051 als PDGF- bzw. Lck-Tyrosin Kinase Inhibitoren, in den Patenten WO9962887 und WO9732858 als Glutamat-Rezeptor Antagonisten beschrieben. In den Literaturstellen J. Med. Chem. 2001, 44, 1758 wird die Synthese von XK469-Derivaten mit Chinoxalinstruktur und deren Verwendung als Antitumormittel, in der Literaturstelle Cancer Research 1996, 3540 Chinoxaline als Flk-1 Inhibitoren bzw. in J. Med. Chem. 1981, 24, 93 die Herstellung von ZNS-aktiven Chinoxalinen beschrieben.

### Darstellung der Erfindung

Die vorliegende Erfindung betrifft Chinoxalin-Derivate der allgemeinen Formel **I,** deren Herstellung sowie deren Verwendung als Arzneimittel, insbesondere zur Behandlung von gutartigen und bösartigen Tumoren am Menschen und Säugetier.

Es wurde jetzt überraschend gefunden, daß insbesondere Chinoxalin-Derivate mit einer Harnstoff- bzw. Thioharnstoffgruppe eine herausragende zytotoxische Wirkung an verschiedenen humanen Tumorzelllinien besitzen. In nanomolaren Konzentrationen wird die Teilung u.a. von Darmkarzinom-, Ovarialkarzinom-, Prostatakarzinom-, Uteruskarzinom-, Glioblastom-, Lungenkarzinom-Zellen, Blutkrebszellen und Brustkrebszellen gehemmt. Die erfinderischen Chinoxaline mit einer Harnstoff- bzw. Thioharnstoffgruppe sind insbesondere auch gegen cis-Platin-, Doxorubicin- und Vincristin-resistente Zelllinien hoch aktiv. Es konnte erfinderisch gezeigt werden, dass die Chinoxaline der allgemeinen Struktur **I** hinsichtlich der biologischen Wirkung hochpotent sind und damit eine Anwendung als Wirkstoff in einem Medikament zur Bekämpfung von Krebserkrankungen möglich ist.

Eine der Erfindung zugrundeliegende Aufgabe ist es nun, zytotoxische Substanzen zur Verfügung zu stellen, die sich zur Behandlung einer Vielzahl von Tumoren, insbesondere bei Wirkstoffresistenzen gegen andere Arzneimittel und bei metastasierenden Karzinomen eignen.

Diese Aufgabe wird gelöst durch Chinoxalin-Derivate der allgemeinen Formel **I** 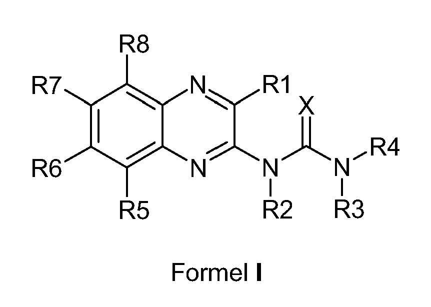 worin
- X:: Sauerstoff oder Schwefel;

- R₁:: (i) Wasserstoff,
(ii) unsubstituiertes oder substituiertes (C₁-C₁₂)-Alkyl,
(iii) Cyano,
(iv) Halogen,
- R₂ /R₃:: (i) Wasserstoff,
(ii) unsubstituiertes oder substituiertes (C₁-C₁₂)-Alkyl,

- R₄:: (i) Wasserstoff,
(ii) unsubstituiertes oder substituiertes (C₁-C₁₂)-Alkyl,
(iii) unsubstituiertes oder substituiertes Cycloalkyl,
(iv) unsubstituiertes oder substituiertes Heterocyclyl,
(v) unsubstituiertes oder substituiertes Aryl,
(vi) unsubstituiertes oder substituiertes Heteroaryl,
(vii) unsubstituiertes oder substituiertes Alkyl-Aryl,
(viii) unsubstituiertes oder substituiertes Alkyl-Heteroaryl,
und

- R₅-R₈:: (i) Wasserstoff,
(ii) unsubstituiertes oder substituiertes (C₁-C₁₂)-Alkyl,
(iii) unsubstituiertes oder substituiertes Aryl,
(iv) unsubstituiertes oder substituiertes Heteroaryl,
(v) Halogen,
(vi) Cyan,
(vii) Hydroxy,
(viii) (C₁-C₁₂)-Alkoxy,
(ix) Amino,
(x) Carboxyl, Alkoxycarbonyl, Carboxy-Alkyl oder Alkoxycarbonyl-Alkyl,
(xi) Alkoxycarbonylamino, Alkoxycarbonylamino-Alkyl bedeuten, und
wobei mindestens einer der Substituenten R₅-R₈ einen unsubstituierten oder substituierten Aryl- oder Heteroaryl-Rest bedeuten muss.

Nachfolgend werden einige Begriffe definiert, die in der Beschreibung und den Patentansprüchen verwendet werden.

Der Ausdruck "Alkyl" umfasst im Sinne dieser Erfindung acyclische gesättigte oder ungesättigte Kohlenwasserstoffreste, die verzweigt oder geradkettig sein können, mit 1 bis 12 C-Atomen, d.h. C₁-₁₂-Alkanyle, C₂-₁₂-Alkenyle und C₂-₁₂-Alkinyle. Dabei weisen Alkenyle mindestens eine C-C-Doppelbindung und Alkinyle mindestens eine C-C-Dreifachbindung auf. Es ist bevorzugt, dass der Alkylrest ausgewählt ist aus der Gruppe, die Methyl, Ethyl, *n*-Propyl, 2-Propyl, *n*-Butyl, *sec*.-Butyl, *tert*.-Butyl, *n*-Pentyl, *iso*-Pentyl, *neo*-Pentyl, *n*-Hexyl, 2-Hexyl, *n*-Octyl, Ethylenyl (Vinyl), Ethinyl, Propenyl (-CH₂CH=CH₂; -CH=CH-CH₃, -C(=CH₂)-CH₃), Propinyl (-CH₂-C≡CH, -C≡C-CH₃), Butenyl, Butinyl, Pentenyl, Pentinyl, Hexenyl, Hexinyl, Heptenyl, Heptinyl, Octenyl und Octinyl enthält.

Der Ausdruck "Cycloalkyl" bedeutet für die Zwecke dieser Erfindung cyclische Kohlenwasserstoffe mit 3-12 Kohlenstoffen, die gesättigt oder ungesättigt sein können. Die Bindung an die Verbindungen der allgemeinen Struktur **I** kann über jedes beliebige und mögliche Ringglied des Cycloalkyl-Restes erfolgen. Der Cycloalkyl-Rest kann auch Teil eines bi- oder polycyclischen Systems sein.

Der Ausdruck "Heterocyclyl" steht für einen 3-, 4-, 5-, 6-, 7- oder 8-gliedrigen cyclischen organischen Rest, der mindestens 1, ggf. 2, 3, 4 oder 5 Heteroatome enthält, wobei die Heteroatome gleich oder verschieden sind und der cyclische Rest gesättigt oder ungesättigt, aber nicht aromatisch ist. Die Bindung an die Verbindungen der allgemeinen Struktur **I** kann über jedes beliebige und mögliche Ringglied des Heterocyclyl-Restes erfolgen. Der Heterocyclus kann auch Teil eines bi- oder polycyclischen Systems sein. Bevorzugte Heteroatome sind Stickstoff, Sauerstoff und Schwefel. Es ist bevorzugt, dass der Heterocyclyl-Rest ausgewählt ist aus der Gruppe, die Tetrahydrofuryl, Tetrahydropyranyl, Pyrrolidinyl, Piperidinyl, Piperazinyl und Morpholinyl enthält.

Der Ausdruck "Aryl" bedeutet im Sinne dieser Erfindung aromatische Kohlenwasserstoffe mit 6 bis 14 C-Atomen, u.a. Phenyle, Naphthyle und Anthracenyle. Die Reste können auch mit weiteren gesättigten, (partiell) ungesättigten oder aromatischen Ringsystemen kondensiert sein. Die Bindung an die Verbindungen der allgemeinen Struktur **I** kann über jedes beliebige und mögliche Ringglied des ArylRestes erfolgen.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome enthält, wobei die Heteroatome gleich oder verschieden sind. Die Bindung an die Verbindungen der allgemeinen Struktur **I** kann über jedes beliebige und mögliche Ringglied des Heteroaryl-Restes erfolgen. Der Heterocyclus kann auch Teil eines bi-oder polycyclischen Systems sein. Bevorzugte Heteroatome sind Stickstoff, Sauerstoff und Schwefel. Es ist bevorzugt, dass der Heteroaryl-Rest ausgewählt ist aus der Gruppe, die Pyrrolyl, Furyl, Thienyl, Thiazolyl, Oxazolyl, Isoxazolyl, Pyrazolyl, Imidazolyl, Pyridinyl, Pyrimidinyl, Pyridazinyl, Pyrazinyl, Phthalazinyl, Indolyl, Indazolyl, Indolizinyl, Chinolinyl, Isochinolinyl, Chinoxalinyl, Chinazolinyl, Carbazolyl, Phenazinyl, Phenothiazinyl, Acridinyl enthält.

Die Ausdrücke "Alkyl-Aryl" oder "Alkyl-Heteroaryl" bedeuten für die Zwecke der vorliegenden Erfindung, daß Alkyl, Aryl und Heteroaryl die oben definierten Bedeutungen haben und der Aryl- oder Heteroaryl-Rest über eine C₁₋₈-Alkyl-Gruppe an die Verbindungen der allgemeinen Struktur **I** gebunden ist.

Im Zusammenhang mit "Alkyl", "Cycloalkyl", "Heterocyclyl", "Aryl", "Heteroaryl", "Alkyl-Aryl" und "Alkyl-Heteroaryl" versteht man unter dem Begriff substituiert im Sinne dieser Erfindung, insofern oben in der Beschreibung oder den Ansprüchen nicht explizit definiert, die Substitution eines oder mehrerer Wasserstoffreste durch F, Cl, Br, I, CN, CF₃, NH₂, NH-Alkyl, NH-Aryl, N(Alkyl)₂, NH-CO-Alkyl, NH-CO-Aryl, NH-CO-Heteroaryl, NH-SO₂-Alkyl, NH-SO₂-Aryl, NH-SO₂-Heteroaryl, NH-CO-NH-Alkyl, NH-CO-NH-Aryl, NH-CO-NH-Heteroaryl, NH-C(O)O-Alkyl, NH-C(O)O-Aryl, NH-C(O)O-Heteroaryl, NO₂, SH, S-Alkyl, OH, OCF₃, O-Alkyl, O-Aryl,O-CO-Alkyl, O-CO-Aryl, O-CO-Heteroaryl, O-C(O)O-Alkyl, O-C(O)O-Aryl, O-C(O)O-Heteroaryl, O-CO-NH-Alkyl, O-CO-N(Alkyl)₂, O-CO-NH-Aryl, O-CO-NH-Heteroaryl, OSO₃H, OSO₂-Alkyl, OSO₂-Aryl, OSO₂-Heteroaryl, OP(O)(OH)₂, Alkyl-P(O)(OH)₂ CHO, CO₂H, C(O)O-Alkyl, C(O)O-Aryl, C(O)O-Heteroaryl, CO-Alkyl, CO-Aryl, CO-Heteroaryl, SO₃H, SO₂-NH₂, SO₂-NH-Alkyl, SO₂-NH-Aryl, SO₂-NH-Heteroaryl, Alkyl, Cycloalkyl, Heterocyclyl, Aryl oder Heteroaryl, wobei die Reste "Alkyl", "Cycloalkyl", "Heterocyclyl", "Aryl" oder "Heteroaryl" ebenfalls substituiert sein können. Die Substituenten können gleich oder verschieden sein und die Substitution kann in jeder beliebigen und möglichen Position des Alkyl-, Cycloalkyl-, Heterocyclyl-, Aryl- und Heteroarylrestes vorkommen.

Unter mehrfach substituierten Resten sind solche zu verstehen, die entweder an verschiedenen oder an gleichen Atomen mehrfach, z. B. zwei- oder dreifach substituiert sind, beispielsweise dreifach am gleichen C-Atom wie im Falle von CF₃, - CH₂CF₃ oder an verschiedenen Stellen wie im Falle von -CH(OH)-CH=CH-CHCl₂.

Die Mehrfachsubstitution kann mit dem gleichen oder verschiedenen Substituenten erfolgen.

Sofern die erfindungsgemäßen Verbindungen der allgemeinen Formel **I** mindestens ein Asymmetriezentrum aufweisen, können sie in Form ihrer Racemate, in Form der reinen Enantiomeren und/oder Diastereomeren oder in Form von Mischungen dieser Enantiomeren und/oder Diastereomeren vorliegen. Die Mischungen können in jedem beliebigen Mischungsverhältnis der Stereoisomeren vorliegen.

So lassen sich beispielsweise die erfindungsgemäßen Verbindungen gemäß der allgemeinen Formel **I,** welche ein oder mehrere Chiralitätszentren aufweisen und die als Racemate auftreten, nach an sich bekannten Methoden in ihre optischen Isomeren, also Enantiomere oder Diastereomere auftrennen. Die Trennung kann durch Säulentrennung an chiralen Phasen oder durch Umkristallisation aus einem optisch aktiven Lösungsmittel oder unter Verwendung einer optisch aktiven Säure oder Base oder durch Derivatisierung mit einem optisch aktiven Reagenz, wie beispielsweise einem optisch aktiven Alkohol, und anschließender Abspaltung des Restes erfolgen.

Sofern möglich, können die erfindungsgemäßen Verbindungen in Form der Tautomeren vorliegen.

Die erfindungsgemäßen Verbindungen der allgemeinen Formel **I** können, falls sie eine ausreichend basische Gruppe, wie zum Beispiel ein primäres, sekundäres oder tertiäres Amin besitzen, mit anorganischen und organischen Säuren in ihre physiologisch verträglichen Salze überführt werden. Vorzugsweise werden die pharmazeutisch annehmbaren Salze der erfindungsgemäßen Verbindungen gemäß der allgemeinen Struktur **I** mit Salzsäure, Bromwasserstoffsäure, Schwefelsäure, Phosphorsäure, Methansulfonsäure, p-Toluolsulfonsäure, Kohlensäure, Ameisensäure, Essigsäure, Trifluoressigsäure, Sulfoessigsäure, Oxalsäure, Malonsäure, Maleinsäure, Bernsteinsäure, Weinsäure, Traubensäure, Äpfelsäure, Embonsäure, Mandelsäure, Fumarsäure, Milchsäure, Citronensäure, Glutaminsäure oder Asparaginsäure gebildet. Bei den gebildeten Salzen handelt es sich u.a. um Hydrochloride, Hydrobromide, Sulfate, Hydrogensulfate, Phosphate, Methansulfonate, Tosylate, Carbonate, Hydrogencarbonate, Formiate, Acetate, Triflate, Sulfoacetate, Oxalate, Malonate, Maleate, Succinate, Tartrate, Malate, Embonate, Mandelate, Fumarate, Lactate, Citrate, Glutaminate und Aspartate. Die Stöchiometrie der gebildeten Salze der erfindungsgemäßen Verbindungen kann dabei ganzzahlige oder nicht ganzzahlige Vielfache von eins betragen.

Die erfindungsgemäßen Verbindungen der allgemeinen Formel **I** können, falls sie eine ausreichend saure Gruppe, wie zum Beispiel die Carboxygruppe enthalten, mit anorganischen und organischen Basen in ihre physiologisch verträglichen Salze überführt werden. Als anorganische Basen kommen beispielsweise Natriumhydroxid, Kaliumhydroxid, Calciumhydroxid, als organische Basen Ethanolamin, Diethanolamin, Triethanolamin, Cyclohexylamin, Dibenzylethylendiamin und Lysin in Betracht. Die Stöchiometrie der gebildeten Salze der erfindungsgemäßen Verbindungen kann dabei ganzzahlige oder nicht ganzzahlige Vielfache von eins betragen.

Ebenfalls bevorzugt sind Solvate und insbesondere Hydrate der erfindungsgemäßen Verbindungen, die z. B. durch Kristallisation aus einem Lösungsmittel oder aus wässriger Lösung erhalten werden können. Es können sich dabei ein, zwei, drei oder beliebig viele Solvat- oder Wasser-Moleküle oder deren ganzzahlige Bruchteile mit den erfindungsgemäßen Verbindungen zu Solvaten und Hydraten verbinden.

Es ist bekannt, dass chemische Substanzen Festkörper ausbilden, die in verschiedenen Ordnungszuständen vorliegen, die man als polymorphe Formen oder Modifikationen bezeichnet. Die verschiedenen Modifikationen einer polymorphen Substanz können sich in ihren physikalischen Eigenschaften stark unterscheiden. Die erfindungsgemäßen Verbindungen der allgemeinen Formel **I** können in verschiedenen polymorphen Formen vorliegen, dabei können bestimmte Modifikationen metastabil sein.

Am meisten bevorzugt sind Verbindungen gemäß der allgemeinen Formel **I,** die in der folgenden Auswahl getroffen sind:
Cyclopentyl-3-[7-(4-hydroxy-3-methoxy-phenyl)-quinoxalin-2-yl]-urea **(1)**
1-Cyclohexyl-3-[7-(4-hydroxy-3-methoxy-phenyl)-quinoxalin-2-yl]-urea **(2)**
Cyclohexyl-3-[7-(3,4-dimethoxy-phenyl)-quinoxalin-2-yl]-urea **(3)**
1-[7-(3,4-Dimethoxy-phenyl)-quinoxalin-2-yl]-3-[1-(2,2,2-trifluoro-acetyl)-piperidin-4-yl]-urea (D-117177) **(4)** 1-[7-(4-Hydroxy-3-methoxy-phenyl)-quinoxalin-2-yl]-3-phenyl-urea **(5)**
1-[7-(4-Hydroxy-3-methoxy-phenyl)-quinoxalin-2-yl]-3-[1-(2,2,2-trifluoro-acety1)-piperidin-4-yl]-urea **(6)**
1-Benzyl-3-[7-(4-hydroxy-3-methoxy-phenyl)-quinoxalin-2-yl]-urea **(7)**
1-Cyclopentyl-3-[7-(3,4-dimethoxy-phenyl)-quinoxalin-2-yl]-urea **(8)**
1-tert-Butyl-3-[7-(4-hydroxy-3,5-dimethyl-phenyl)-quinoxalin-2-yl]-urea **(9)**
1-tert-Butyl-3-[7-(4-hydroxy-3-methoxy-phenyl)-quinoxalin-2-yl]-urea **(10)**
1-[7-(4-Hydroxy-3-methoxy-phenyl)-quinoxalin-2-yl]-3-thiophen-2-yl-urea **(11)**
1-Cyclohexyl-3-[7-(4-hydroxy-3,5-dimethyl-phenyl)-quinoxalin-2-yl]-urea **(12)**
1-Cyclopentyl-3-[7-(4-hydroxy-3,5-dimethyl-phenyl)-quinoxalin-2-yl]-urea **(13)**
1-[7-(4-Hydroxy-3,5-dimethyl-phenyl)-quinoxalin-2-yl]-3-phenyl-urea **(14)**
1-[7-(4-Hydroxy-3,5-dimethyl-phenyl)-quinoxalin-2-yl]-3-thiophen-2-yl-urea **(15)**
1-[7-(4-Hydroxy-3,5-dimethyl-phenyl)-quinoxalin-2-yl]-3-methyl-urea **(16)**
1-[7-(4-Hydroxy-3,5-dimethyl-phenyl)-quinoxalin-2-yl]-3-isopropyl-urea **(17)**
1-Butyl-3-[7-(4-hydroxy-3,5-dimethyl-phenyl)-quinoxalin-2-yl]-urea **(18)**
1-Ethyl-3-[7-(4-hydroxy-3,5-dimethyl-phenyl)-quinoxalin-2-yl]-urea **(19)**
1-Cyclohexyl-3-[7-(4-hydroxy-3,5-dimethyl-phenyl)-quinoxalin-2-yl]-thiourea **(20)**
1-Cyclohexyl-3-[7-(3,5-dichloro-4-hydroxy-phenyl)-quinoxalin-2-yl]-thiourea **(21)**
1-Ethyl-3-[7-(4-hydroxy-3,5-dimethyl-phenyl)-quinoxalin-2-yl]-thiourea **(22)**
1-Cyclopropyl-3-[7-(4-hydroxy-3,5-dimethyl-phenyl)-quinoxalin-2-yl]-thiourea **(23)**
1-Cyclopentyl-3-[7-(4-hydroxy-3,5-dimethyl-phenyl)-quinoxalin-2-yl]-thiourea **(24)**
1-tert-Butyl-3-[7-(4-hydroxy-3,5-dimethyl-phenyl)-quinoxalin-2-yl]-thiourea **(25)**
1-Dodecyl-3-(7-phenyl-quinoxalin-2-yl)-urea **(26)**
1-(3-Chloro-2-methyl-phenyl)-3-(7-phenyl-quinoxalin-2-yl)-urea **(27)**
1-(3,4-Dimethyl-phenyl)-3-(7-phenyl-quinoxalin-2-yl)-urea **(28)**
1-Allyl-3-(7-phenyl-quinoxalin-2-yl)-urea **(29)**
1-Cyclopentyl-3-(7-phenyl-quinoxalin-2-yl)-urea **(30)**
1-[7-(4-Hydroxy-3,5-dimethyl-phenyl)-quinoxalin-2-yl]-3-isopropyl-thiourea **(31)**
1-Butyl-3-[7-(4-hydroxy-3,5-dimethyl-phenyl)-quinoxalin-2-yl]-thiourea **(32)**
1-[7-(4-Hydroxy-3,5-dimethyl-phenyl)-quinoxalin-2-yl]-3-pentyl-urea **(33)**
1-Cyclobutyl-3-[7-(4-hydroxy-3,5-dimethyl-phenyl)-quinoxalin-2-yl]-thiourea **(34)**
1-Hexyl-3-[7-(4-hydroxy-3,5-dimethyl-phenyl)-quinoxalin-2-yl]-urea **(35)**
1-[7-(4-Hydroxy-3,5-dimethyl-phenyl)-quinoxalin-2-yl]-3-propyl-urea **(36)**
1-Dodecyl-3-[7-(4-hydroxy-3,5-dimethyl-phenyl)-quinoxalin-2-yl]-urea **(37)**
1-(3-Chloro-2-methyl-phenyl)-3-[7-(4-hydroxy-3,5-dimethyl-phenyl)-quinoxalin-2-yl]-urea **(38)**
1-(3-Acetyl-phenyl)-3-[7-(4-hydroxy-3,5-dimethyl-phenyl)-quinoxalin-2-yl]-urea **(39)**
1-(3,4-Dimethyl-phenyl)-3-[7-(4-hydroxy-3,5-dimethyl-phenyl)-quinoxalin-2-yl]-urea **(40)**
1-Allyl-3-[7-(4-hydroxy-3,5-dimethyl-phenyl)-quinoxalin-2-yl]-urea **(41)**
1-Cyclooctyl-3-[7-(4-hydroxy-3,5-dimethyl-phenyl)-quinoxalin-2-yl]-urea **(42)**
1-(2,4-Dimethyl-phenyl)-3-[7-(4-hydroxy-3,5-dimethyl-phenyl)-quinoxalin-2-yl]-urea **(43)**
1-Cyclooctyl-3-[7-(4-hydroxy-3-methoxy-phenyl)-quinoxalin-2-yl]-urea **(44)**
1-Adamantan-1-yl-3-(7-phenyl-quinoxalin-2-yl)-urea **(45)**
1-[7-(4-Hydroxy-3,5-dimethyl-phenyl)-quinoxalin-2-yl]-3-p-tolyl-urea **(46)**
1-(3,4-Dichloro-phenyl)-3-[7-(4-hydroxy-3,5-dimethyl-phenyl)-quinoxalin-2-yl]-urea **(47)**
1-[7-(4-Hydroxy-3,5-dimethyl-phenyl)-quinoxalin-2-yl]-3-(4-methoxy-phenyl)-urea **(48)**
1-Adamantan-1-yl-3-[7-(4-hydroxy-3,5-dimethyl-phenyl)-quinoxalin-2-yl]-urea **(49)**
1-[7-(4-Hydroxy-3,5-dimethyl-phenyl)-quinoxalin-2-yl]-3-naphthalen-2-yl-urea **(50)**
1-[7-(4-Hydroxy-3,5-dimethyl-phenyl)-quinoxalin-2-yl]-3-(1,1,3,3-tetramethyl-butyl)-urea **(51)**
1-[7-(4-Hydroxy-3,5-dimethyl-phenyl)-quinoxalin-2-yl]-3-pyridin-3-yl-urea **(52)**
1-((R)-1,2-Dimethyl-propyl)-3-[7-(4-hydroxy-3,5-dimethyl-phenyl)-quinoxalin-2-yl]-urea **(53)**
1-((S)-1,2-Dimethyl-propyl)-3-[7-(4-hydroxy-3,5-dimethyl-phenyl)-quinoxalin-2-yl]-urea **(54)**
1-(5-Chloro-2-methyl-phenyl)-3-[7-(4-hydroxy-3,5-dimethyl-phenyl)-quinoxalin-2-yl]-urea **(55)**
1-((S)-2-Phenyl-cyclopropyl)-3-(7-phenyl-quinoxalin-2-yl)-urea **(56)**
1-[7-(4-Hydroxy-3,5-dimethyl-phenyl)-quinoxalin-2-yl]-3-((S)-2-phenyl-cyclopropyl)-urea **(57)**
1-(2-Chloro-6-methyl-phenyl)-3-[7-(4-hydroxy-3,5-dimethyl-phenyl)-quinoxalin-2-yl]-urea **(58)**
1-Allyl-3-[7-(4-hydroxy-3-methoxy-phenyl)-quinoxalin-2-yl]-urea (D-118068) **(59)**
1-(3,5-Dimethyl-isoxazol-4-yl)-3-[7-(4-hydroxy-3,5-dimethyl-phenyl)-quinoxalin-2-yl]-urea **(60)**
1-sec-Butyl-3-[7-(4-hydroxy-3,5-dimethyl-phenyl)-quinoxalin-2-yl]-urea **(61)**
1-Ethyl-3-[7-(4-methoxy-3,5-dimethyl-phenyl)-quinoxalin-2-yl]-urea **(62)**
1-Isopropyl-3-[7-(4-methoxy-3,5-dimethyl-phenyl)-quinoxalin-2-yl]-urea **(63)**
1-Isopropyl-3-[7-(4-methoxy-3,5-dimethyl-phenyl)-quinoxalin-2-yl]-thiourea **(64)**
1-[7-(4-Hydroxy-3,5-dimethyl-phenyl)-quinoxalin-2-yl]-3-isobutyl-urea **(65)**
1-(1-Ethyl-propyl)-3-[7-(4-hydroxy-3,5-dimethyl-phenyl)-quinoxalin-2-yl]-urea **(66)**

Die erfindungsgemäßen Chinoxaline gemäß der allgemeinen Formel **I** sind als Wirkstoffe in Arzneimitteln, insbesondere als Antitumormittel, zur Behandlung von Menschen und Säugetieren geeignet. Säugetiere können Haustiere wie Pferde, Kühe, Hunde, Katzen, Hasen, Schafe und dergleichen sein.

Gemäß einem weiteren Aspekt der Erfindung wird ein Verfahren zur Behandlung von Tumoren beim Menschen und in Säugetieren bereit gestellt, welches dadurch gekennzeichnet ist, daß mindestens ein Chinoxalin gemäß der allgemeinen Formel **I** dem Menschen oder einem Säugetier in einer für die Tumorbehandlung wirksamen Menge verabreicht wird. Die für die Behandlung zu verabreichende therapeutisch effektive Dosis des jeweiligen erfindungsgemäßen Chinoxalins richtet sich u.a. nach der Art und dem Stadium der Tumorerkrankung, dem Alter, Gewicht und Geschlecht des Patienten, der Art der Verabreichung und der Dauer der Behandlung. Die erfindungsgemäßen Arzneimittel können als flüssige, halbfeste und feste Arzneiformen verabreicht werden. Dies erfolgt in der jeweils geeigneten Weise in Form von Aerosolen, Pulver, Puder und Streupuder, Tabletten, Dragees, Emulsionen, Schäume, Lösungen, Suspensionen, Gele, Salben, Pasten, Pillen, Pastillen, Kapseln oder Suppositorien.

Die Arzneiformen enthalten neben mindestens einem erfindungsgemäßen Bestandteil je nach eingesetzter galenischer Form gegebenenfalls Hilfsstoffe, wie unter anderem Lösungsmittel, Lösungsbeschleuniger, Lösungsvermittler, Emulgatoren, Netzmittel, Antischaummittel, Gelbildner, Verdickungsmittel, Filmbildner, Bindemittel, Puffer, Salzbildner, Trocknungsmittel, Fließregulierungsmittel, Füllstoffe, Konservierungsstoffe, Antioxidatien, Farbstoffe, Formentrennmittel, Gleitmittel, Sprengmittel, Geschmacks-und Geruchskorrigentien. Die Auswahl der Hilfsstoffe sowie die einzusetzenden Mengen derselben hängt von der gewählten galenischen Form ab und orientiert sich an die dem Fachmann bekannten Rezepturen.

Die erfindungsgemäßen Arzneimittel können in einer geeigneten Darreichungsform auf die Haut, epicutan als Lösung, Suspension, Emulsion, Schaum, Salbe, Paste oder Pflaster; über die Mund- und Zungenschleimhaut, buccal, lingual oder sublingual als Tablette, Pastille, Dragees, Linctus oder Gurgelwasser; über die Magen- und Darmschleimhaut, enteral als Tablette, Dragees, Kapsel, Lösung, Suspension oder Emulsion; über die Rectumschleimhaut, rectal als Suppositorium, Rectalkapsel oder Salbe; über die Nasenschleimhaut, nasal als Tropfen, Salben oder Spray; über das Bronchial- und Alveolarepithel, pulmonal oder per inhalationem als Aerosol oder Inhalat; über die Conjunctiva, conjunctival als Augentropfen, Augensalbe, Augentabletten, Lamellae oder Augenwasser; über die Schleimhäute der Genitalorgane, intravaginal als Vaginalkugeln, Salben und Spülung, intrauterin als Uterus-Pessare; über die ableitenden Harnwege, intraurethral als Spülung, Salbe oder Arzneistäbchen; in eine Arterie, intraarteriell als Injektion; in eine Vene, intravenös als Injektion oder Infusion, paravenös als Injektion oder Infusion; in die Haut, intracutan als Injektion oder Implantat; unter die Haut, subcutan als Injektion oder Implantat; in den Muskel, intramusculär als Injektion oder Implantat; in die Bauchhöhle, intraperitoneal als Injektion oder Infusion verabreicht werden.

Die erfindungsgemässen Verbindungen der allgemeinen Struktur **I** können in Hinblick auf praktische therapeutische Erfordernisse mittels geeigneter Maßnahmen in ihrer Arzneistoffwirkung verlängert werden. Dieses Ziel kann auf chemischem und/oder galenischem Wege erreicht werden. Beispiele für die Erzielung einer Wirkungsverlängerung sind der Einsatz von Implantaten, Liposomen, Retardformen, Nanopartikelsuspensionen und so genannter Prodrugs der erfindungsgemäßen Verbindungen, die Bildung von schwerlöslichen Salzen und Komplexen oder der Einsatz von Kristall-Suspensionen.

Die erfindungsgemäßen Verbindungen können als Einzelsubstanz oder in Kombination mit weiteren Substanzen, wie z.B. Asparaginase, Bleomycin, Carboplatin, Carmustine, Chlorambucil, Cisplatin, Colaspase, Cyclophosphamide, Cytarabine, Dacarbazine, Dactinomycin, Daunorubicin, Doxorubicin (Adriamycin), Epirubicin, Etoposide, 5-Fluorouracil, Hexamethylmelamine, Hydroxyurea, Ifosfamide, Irinotecan, Leucovorin, Lomustine, Mechlorethamine, 6-Mercaptopurine, Mesna, Methotrexate, Mitomycin C, Mitoxantrone, Prednisolone, Prednisone, Procarbazine, Raloxifen, Streptozocin, Tamoxifen, Thalidomide, Thioguanine, Topotecan, Vinblastine, Vincristine, Vindesine, Aminoglutethimide, L-Asparaginase, Azathioprine, 5-Azacytidine cladribine, Busulfan, Diethylstilbestrol, 2',2'-Difluorodeoxycytidine, Docetaxel, Erythrohydroxynonyladenine, Ethynylestradiol, 5-Fluorodeoxyuridine, 5-Fluorodeoxyuridine monophosphate, Fludarabine phosphate, Fluoxymesterone, Flutamide, Hydroxyprogesterone caproate, Idarubicin, Interferon, Medroxyprogesterone acetate, Megestrol acetate, Melphalan, Mitotane, Paclitaxel, Oxaliplatin, Pentostatin, N-Phosphonoacetyl-L-aspartate (PALA), Plicamycin, Semustine, Teniposide, Testosterone propionate, Thiotepa, Trimethylmelamine, Uridine, Vinorelbine, Epothilone, Gemcitabine, Taxotere, BCNU, CCNU, DTIC, Herceptin, Avastin, Erbitux, Sorafenib, Gleevec, Iressa, Tarceva, Rapamycin, Actinomycin D, Sunitinib (sutent) eingesetzt werden.

Besonders bevorzugt sind dabei Arzneimittel, die mindestens eine Verbindung aus der nachfolgenden Gruppe der Chinoxaline enthalten:
Cyclopentyl-3-[7-(4-hydroxy-3-methoxy-phenyl)-quinoxalin-2-yl]-urea **(1)**
1-Cyclohexyl-3-[7-(4-hydroxy-3-methoxy-phenyl)-quinoxalin-2-yl]-urea **(2)**
Cyclohexyl-3-[7-(3,4-dimethoxy-phenyl)-quinoxalin-2-yl]-urea **(3)** 1-[7-(3,4-Dimethoxy-phenyl)-quinoxalin-2-yl]-3-[1-(2,2,2-trifluoro-acetyl)-piperidin-4-yl]-urea (D-117177) **(4)**
1-[7-(4-Hydroxy-3-methoxy-phenyl)-quinoxalin-2-yl]-3-phenyl-urea **(5)**
1-[7-(4-Hydroxy-3-methoxy-phenyl)-quinoxalin-2-yl]-3-[1-(2,2,2-trifluoro-acetyl)-piperidin-4-yl]-urea **(6)**
1-Benzyl-3-[7-(4-hydroxy-3-methoxy-phenyl)-quinoxalin-2-yl]-urea **(7)**
1-Cyclopentyl-3-[7-(3,4-dimethoxy-phenyl)-quinoxalin-2-yl]-urea **(8)**
1-tert-Butyl-3-[7-(4-hydroxy-3,5-dimethyl-phenyl)-quinoxalin-2-yl]-urea **(9)**
1-tert-Butyl-3-[7-(4-hydroxy-3-methoxy-phenyl)-quinoxalin-2-yl]-urea **(10)**
1-[7-(4-Hydroxy-3-methoxy-phenyl)-quinoxalin-2-yl]-3-thiophen-2-yl-urea **(11)**
1-Cyclohexyl-3-[7-(4-hydroxy-3,5-dimethyl-phenyl)-quinoxalin-2-yl]-urea **(12)**
1-Cyclopentyl-3-[7-(4-hydroxy-3,5-dimethyl-phenyl)-quinoxalin-2-yl]-urea **(13)**
1-[7-(4-Hydroxy-3,5-dimethyl-phenyl)-quinoxalin-2-yl]-3-phenyl-urea **(14)**
1-[7-(4-Hydroxy-3,5-dimethyl-phenyl)-quinoxalin-2-yl]-3-thiophen-2-yl-urea **(15)**
1-[7-(4-Hydroxy-3,5-dimethyl-phenyl)-quinoxalin-2-yl]-3-methyl-urea **(16)**
1-[7-(4-Hydroxy-3,5-dimethyl-phenyl)-quinoxalin-2-yl]-3-isopropyl-urea **(17)**
1-Butyl-3-[7-(4-hydroxy-3,5-dimethyl-phenyl)-quinoxalin-2-yl]-urea **(18)**
1-Ethyl-3-[7-(4-hydroxy-3,5-dimethyl-phenyl)-quinoxalin-2-yl]-urea **(19)**
1-Cyclohexyl-3-[7-(4-hydroxy-3,5-dimethyl-phenyl)-quinoxalin-2-yl]-thiourea **(20)**
1-Cyclohexyl-3-[7-(3,5-dichloro-4-hydroxy-phenyl)-quinoxalin-2-yl]-thiourea **(21)**
1-Ethyl-3-[7-(4-hydroxy-3,5-dimethyl-phenyl)-quinoxalin-2-yl]-thiourea **(22)**
1-Cyclopropyl-3-[7-(4-hydroxy-3,5-dimethyl-phenyl)-quinoxalin-2-yl]-thiourea **(23)**
1-Cyclopentyl-3-[7-(4-hydroxy-3,5-dimethyl-phenyl)-quinoxalin-2-yl]-thiourea **(24)**
1-tert-Butyl-3-[7-(4-hydroxy-3,5-dimethyl-phenyl)-quinoxalin-2-yl]-thiourea **(25)**
1-Dodecyl-3-(7-phenyl-quinoxalin-2-yl)-urea **(26)**
1-(3-Chloro-2-methyl-phenyl)-3-(7-phenyl-quinoxalin-2-yl)-urea **(27)**
1-(3,4-Dimethyl-phenyl)-3-(7-phenyl-quinoxalin-2-yl)-urea **(28)**
1-Allyl-3-(7-phenyl-quinoxalin-2-yl)-urea **(29)**
1-Cyclopentyl-3-(7-phenyl-quinoxalin-2-yl)-urea **(30)**
1-[7-(4-Hydroxy-3,5-dimethyl-phenyl)-quinoxalin-2-yl]-3-isopropyl-thiourea **(31)**
1-Butyl-3-[7-(4-hydroxy-3,5-dimethyl-phenyl)-quinoxalin-2-yl]-thiourea **(32)**
1-[7-(4-Hydroxy-3,5-dimethyl-phenyl)-quinoxalin-2-yl]-3-pentyl-urea **(33)**
1-Cyclobutyl-3-[7-(4-hydroxy-3,5-dimethyl-phenyl)-quinoxalin-2-yl]-thiourea **(34)**
1-Hexyl-3-[7-(4-hydroxy-3,5-dimethyl-phenyl)-quinoxalin-2-yl]-urea **(35)**
1-[7-(4-Hydroxy-3,5-dimethyl-phenyl)-quinoxalin-2-yl]-3-propyl-urea **(36)**
1-Dodecyl-3-[7-(4-hydroxy-3,5-dimethyl-phenyl)-quinoxalin-2-yl]-urea **(37)**
1-(3-Chloro-2-methyl-phenyl)-3-[7-(4-hydroxy-3,5-dimethyl-phenyl)-quinoxalin-2-yl]-urea **(38)**
1-(3-Acetyl-phenyl)-3-[7-(4-hydroxy-3,5-dimethyl-phenyl)-quinoxalin-2-yl]-urea **(39)**
1-(3,4-Dimethyl-phenyl)-3-[7-(4-hydroxy-3,5-dimethyl-phenyl)-quinoxalin-2-yl]-urea **(40)**
1-Allyl-3-[7-(4-hydroxy-3,5-dimethyl-phenyl)-quinoxalin-2-yl]-urea **(41)**
1-Cyclooctyl-3-[7-(4-hydroxy-3,5-dimethyl-phenyl)-quinoxalin-2-yl]-urea **(42)**
1-(2,4-Dimethyl-phenyl)-3-[7-(4-hydroxy-3,5-dimethyl-phenyl)-quinoxalin-2-yl]-urea **(43)**
1-Cyclooctyl-3-[7-(4-hydroxy-3-methoxy-phenyl)-quinoxalin-2-yl]-urea **(44)**
1-Adamantan-1-yl-3-(7-phenyl-quinoxalin-2-yl)-urea **(45)**
1-[7-(4-Hydroxy-3,5-dimethyl-phenyl)-quinoxalin-2-yl]-3-p-tolyl-urea **(46)**
1-(3,4-Dichloro-phenyl)-3-[7-(4-hydroxy-3,5-dimethyl-phenyl)-quinoxalin-2-yl]-urea **(47)**
1-[7-(4-Hydroxy-3,5-dimethyl-phenyl)-quinoxalin-2-yl]-3-(4-methoxy-phenyl)-urea **(48)**
1-Adamantan-1-yl-3-[7-(4-hydroxy-3,5-dimethyl-phenyl)-quinoxalin-2-yl]-urea **(49)**
1-[7-(4-Hydroxy-3,5-dimethyl-phenyl)-quinoxalin-2-yl]-3-naphthalen-2-yl-urea **(50)**
1-[7-(4-Hydroxy-3,5-dimethyl-phenyl)-quinoxalin-2-yl]-3-(1,1,3,3-tetramethyl-butyl)-urea **(51)**
1-[7-(4-Hydroxy-3,5-dimethyl-phenyl)-quinoxalin-2-yl]-3-pyridin-3-yl-urea **(52)**
1-((R)-1,2-Dimethyl-propyl)-3-[7-(4-hydroxy-3,5-dimethyl-phenyl)-quinoxalin-2-yl]-urea **(53)**
1-((S)-1,2-Dimethyl-propyl)-3-[7-(4-hydroxy-3,5-dimethyl-phenyl)-quinoxalin-2-yl]-urea **(54)**
1-(5-Chloro-2-methyl-phenyl)-3-[7-(4-hydroxy-3,5-dimethyl-phenyl)-quinoxalin-2-yl]-urea **(55)**
1-((S)-2-Phenyl-cyclopropyl)-3-(7-phenyl-quinoxalin-2-yl)-urea **(56)**
1-[7-(4-Hydroxy-3,5-dimethyl-phenyl)-quinoxalin-2-yl]-3-((S)-2-phenyl-cyclopropyl)-urea **(57)**
1-(2-Chloro-6-methyl-phenyl)-3-[7-(4-hydroxy-3,5-dimethyl-phenyl)-quinoxalin-2-yl]-urea **(58)**
1-Allyl-3-[7-(4-hydroxy-3-methoxy-phenyl)-quinoxalin-2-yl]-urea (D-118068) **(59)**
1-(3,5-Dimethyl-isoxazol-4-yl)-3-[7-(4-hydroxy-3,5-dimethyl-phenyl)-quinoxalin-2-yl]-urea **(60)**
1-sec-Butyl-3-[7-(4-hydroxy-3,5-dimethyl-phenyl)-quinoxalin-2-yl]-urea **(61)**
1-Ethyl-3-[7-(4-methoxy-3,5-dimethyl-phenyl)-quinoxalin-2-yl]-urea **(62)**
1-Isopropyl-3-[7-(4-methoxy-3,5-dimethyl-phenyl)-quinoxalin-2-yl]-urea **(63)**
1-Isopropyl-3-[7-(4-methoxy-3,5-dimethyl-phenyl)-quinoxalin-2-yl]-thiourea **(64)**
1-[7-(4-Hydroxy-3,5-dimethyl-phenyl)-quinoxalin-2-yl]-3-isobutyl-urea **(65)**
1-(1-Ethyl-propyl)-3-[7-(4-hydroxy-3,5-dimethyl-phenyl)-quinoxalin-2-yl]-urea **(66)**

Diese Verbindungen können sowohl als freie Base als auch als Salze physiologisch verträglicher Säuren vorliegen.

### Chemische Synthese

Die Verbindungen der allgemeinen Formel **I** sind gemäß dem folgenden Schema 1 erhältlich:

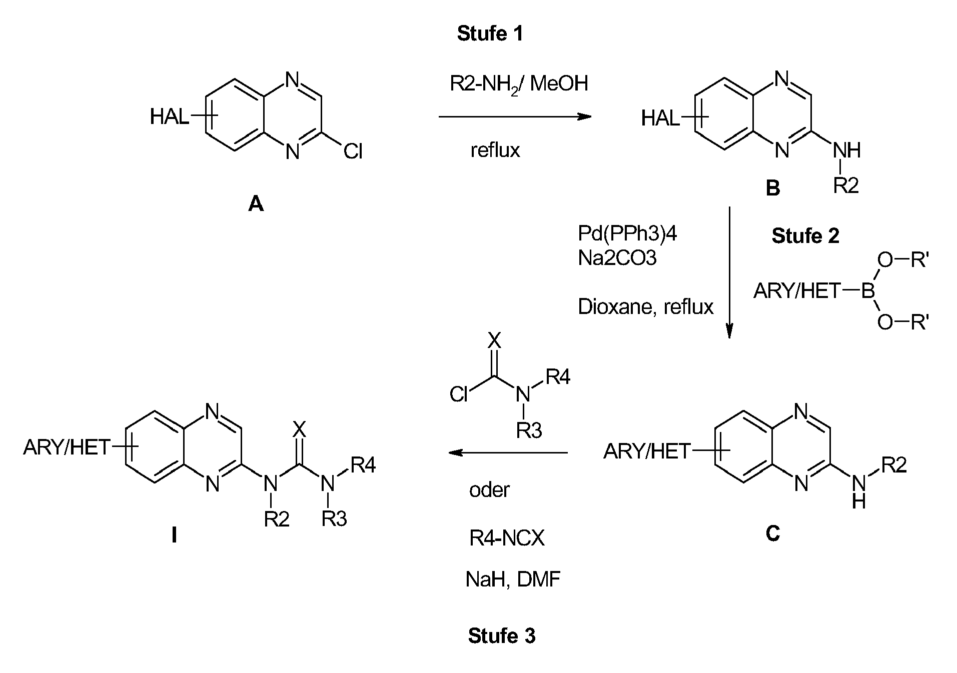

### Schema 1

Die Ausgangsverbindungen sind entweder im Handel erhältlich oder können nach an sich bekannten Verfahrensweisen hergestellt werden. Die Edukte B und C stellen wertvolle Zwischenverbindungen für die Herstellung der erfindungsgemäßen Chinoxaline der allgemeinen Formel **I** dar.

Für die Herstellung der Ausgangs- und Zielverbindungen sei beispielsweise auf folgende Primärliteratur verwiesen, deren Inhalt hiermit Bestandteil der Offenbarung der vorliegenden Anmeldung werden soll:
1) W.C. Lumma et al. J. Med. Chem. 1991, 24, 93-101;
2) J.P. Horwitz et al. J. Med. Chem. 2001, 44, 1758-1776.

Die gegebenenfalls zu verwendenden Lösungs- und Hilfsmittel und anzuwendenden Reaktionsparameter wie Reaktionstemperatur und -dauer sind dem Fachmann aufgrund seines Fachwissens bekannt.

Gemäß dieser allgemeinen Vorschrift für die Stufen 1, 2 und 3, denen das Syntheseschema 1 zugrunde liegt, wurden folgende Verbindungen synthetisiert, die unter der Angabe der jeweiligen chemischen Bezeichnung aus der nachfolgenden Übersicht hervorgehen. Die analytische Charakterisierung der erfindungsgemäßen Verbindungen erfolgte durch ihre Schmelzpunkte bzw. ¹H-NMR-spektroskopisch und/oder massenspektroskopisch.

Die eingesetzten Chemikalien und Lösungsmittel wurden kommerziell bei den herkömmlichen Anbietern erworben (Acros, Avocado, Aldrich, Fluka, Lancaster, Maybridge, Merck, Sigma, TCI etc.) oder synthetisiert.

### Beispiele

Die Erfindung soll anhand der nachfolgenden Beispiele näher erläutert werden, ohne darauf beschränkt zu sein.

Die chemische Benennung der Substanzen erfolgte mit der AutoNom 2000 Software (ISIS ™/ Draw 2.5 SP2; MDL).

### Beispiel 1 (Umsetzung gemäß Schema 1, 1. Stufe):

### Beispiel 1.1: 7-Chlor-chinoxalin-2-ylamin

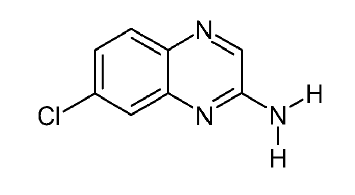

30.0g (60.0 mmol) 2,7-Dichlorchinoxalin werden in 400ml NH₃/MeOH (w= 22%) 16 Stunden bei T=120°C gerührt. Im Anschluss wird der Ansatz bis zur Trockene im Vakuum aufkonzentriert und der Rückstand in 250ml Dichlormethan und 250ml Wasser aufgenommen. Nach Phasentrennung wird die organische Phase nachfolgend mit 250 ml Wasser gewaschen, über Natriumsulfat getrocknet und das Lösungsmittel im Membranpumpenvakuum abgezogen. Der erhaltene Rückstand wird säulenchromatographisch an Kieselgel (Essigsäureethylester/Petrolether= 1:5; Silicagel: 200-300 mesh) aufgereinigt, wobei 10.5g an 7-Chlor-chinoxalin-2-ylamin resultieren (Ausbeute 98%).
m.p.: 219-222°C
¹HNMR (300MHz, CDCl₃) δ= 8.30 (1H, s), 7.83 (1H, s), 7.65 (1H, m), 7.40 (1H, m) ppm MS (ESI) m/z 180 (MH⁺)

### Beispiel 2 (Umsetzung gemäß Schema 1, 2. Stufe)

### Beispiel 2.1: 4-(3-Amino-chinoxalin-6-yl)-2,6-dimethyl-phenol

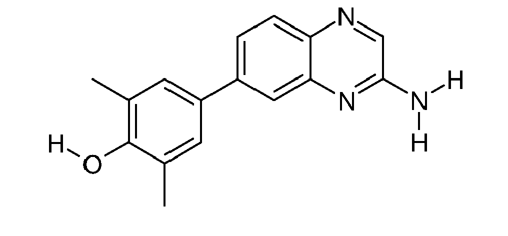

Zu einer Lösung von 0.8g (4.5 mmol) 7-Chlor-chinoxalin-2-ylamin in 40ml Dioxan werden unter Argon 1.66g (6.7 mmol) 3,5-Dimethyl-4-hydroxyphenylboronsäurepinacolester, 1.42g (13.4 mmol) Natriumcarbonat gelöst in 10 ml Wasser, und 0.155 g (0.13 mmol) Pd(PPh₃)₄ zugegeben und für 4 Stunden bei 100 °C gerührt. Zur Aufarbeitung wird das Reaktionsgemisch mit 30 mL Essigsäureethylester und 30 mL Wasser versetzt, die Phasen getrennt, die organische Phase über Magnesiumsulfat getrocknet, filtriert und das Lösungsmittel am Rotationsverdampfer entfernt. Der hierbei erhaltene Rückstand wird säulenchromatographisch an Kieselgel (Essigester/nHeptan)
aufgereinigt, wobei 0.65g an 4-(3-Amino-chinoxalin-6-yl)-2,6-dimethyl-phenol isoliert werden.
Ausbeute: 33.1 %
m.p.: 256°C
¹HNMR (600MHz, DMSO-*d₆*) δ= 8.43 (1H, br. s ), 8.24 (1H, s), 7.75 (1H, d), 7.62 (1H, m), 7.57 (1H, m), 7.35 (2H, s), 6.90 (2H, s), 2.25 (6H, s) ppm
MS (ESI) m/z 266 (MH⁺)

Folgende Zwischenstufen der Formel B wurden analog zum Beispiel 2.1 (4-(3-Aminochinoxalin-6-yl)-2,6-dimethyl-phenol) synthetisiert.

### Bsp 2.2: 7-Phenylchinoxalin-2-amin

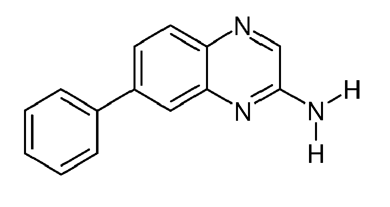

¹HNMR (300MHz, DMSO-*d₆*) δ= 8.93 (1H, m), 8.81 (1H, s), 8.00 (5H, m), 7.45 (3H, s) ppm
MS (ESI) m/z 222 (MH⁺)

### Bsp 2.3: 4-(3-Aminochinoxalin-6-yl)-2-methoxyphenol

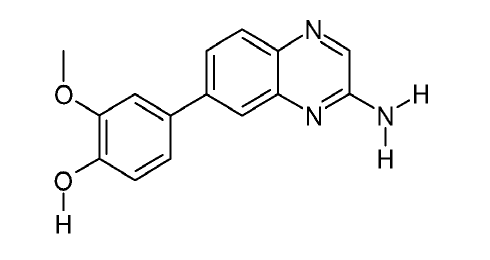

¹HNMR (300MHz, DMSO-*d₆*) δ= 8.26 (1H, s), 7.70 (4H, s), 7.30 (2 H, J=5.4 Hz, m), 6.90 (1H, m), 4.00 (3H, s) ppm
MS (ESI) 269 (MH⁺)

### Bsp 2.4: 7-(3, 4-Dimethoxyphenyl)chinoxalin-2-amin

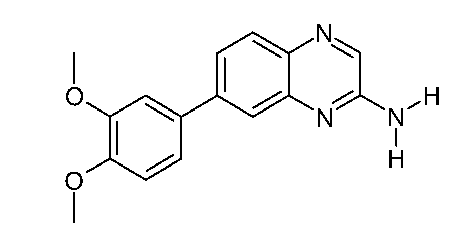

¹HNMR (300MHz, DMSO-*d₆*) δ= 8.26 (1H, s), 7.50 (4H, s), 7.30 (1H, J=5.4 Hz, m), 6.90 (2H, m), 3.88 (6H, s) ppm;
MS (ESI) 282 (MH⁺)

### Bsp 2.5: 4-(3-Amino-chinoxalin-6-yl)-2,6-dichlor-phenol

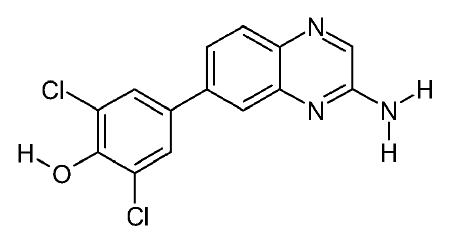

m.p.: 263°C
¹HNMR (600MHz, DMSO-*d₆*) δ= 10.35 (1H, s), 8.28 (1H, s), 7.78 (3H, m), 7.71 (1H, m), 7.63 (1H, m), 7.02 (2H, s) ppm
MS (ESI) m/z 306 (MH⁺)

### Bsp 2.6: 7-(4-Methoxy-3,5-dimethyl-phenyl)-chinoxalin-2-ylamin

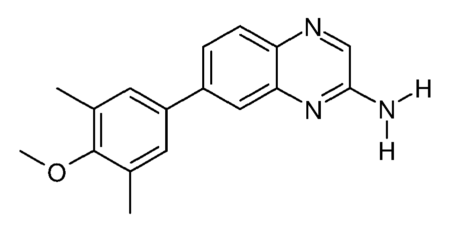

¹HNMR (600MHz, DMSO-*d₆*) δ= 8.26 (1H, s), 7.78 (1H, d), 7.67 (1H, m), 7.58 (1H, m), 7.43 (2H, s), 6.95 (2H, s), 3.75 (3H, s) ppm
MS (ESI) m/z 280 (MH⁺)

### Beispiel 3 (Umsetzung gemäß Schema 1, 3. Stufe)

### Allgemeine Arbeitsvorschrift (AAV) zur Herstellung der Harnstoffe bzw. Thioharnstoffe:

Zu einer Mischung von 0.71 mmol Natriumhydrid (60%ige Mineralölsuspension) in 5 ml Dimethylformamid oder THF werden unter Eiskühlung 0.45 mmol Zwischenstufe B (s. Beispiel 2) hinzugegeben. Nach 30 min Rühren bei 0°C werden 0.54 mmol des entsprechenden Isocyanats bzw. Isothiocyanats gelöst in 2 ml THF tropfenweise hinzugegeben und die Eiskühlung entfernt. Nach 16 h Rühren bei Raumtemperatur wird die Reaktionslösung im Membranpumpenvakuum bis zur Trockene aufkonzentriert und der Rückstand in 50ml Wasser und 50ml Essigester aufgenommen, wobei das Produkt präzipitiert. Absaugen des Niederschlages und anschließende Trocknung im Vakuumtrockenschrank führt zu der Zielverbindung.

Folgende Verbindungen der Formel **I** wurden analog zum Syntheseweg in Schema 1 und gemäß der allgemeinen Arbeitsvorschrift synthetisiert:

### Beispiel 3.1: Cyclopentyl-3-[7-(4-hydroxy-3-methoxy-phenyl)-quinoxalin-2-yl]-urea (1)

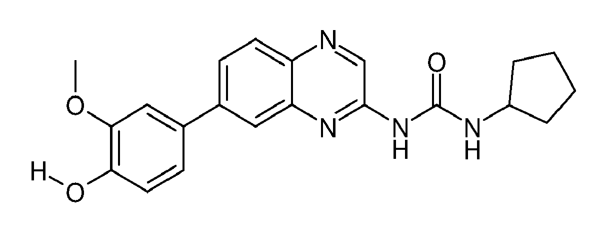

¹HNMR (300MHz, DMSO-*d₆*) δ= 9.99 (1H, s), 8.80 (1H, m), 8.75 (1H, s), 7.95 (3H, s), 7.25 (2H, s), 6.90 (1H, m), 4.01 (1H, m), 3.90 (3H, s),1.90 (3H, s),1.6 (5H, s) ppm
MS (ESI) m/z 379 (MH⁺)

### Beispiel 3.2 :1-Cyclohexyl-3-[7-(4-hydroxy-3-methoxy-phenyl)-quinoxalin-2-yl]-urea (2)

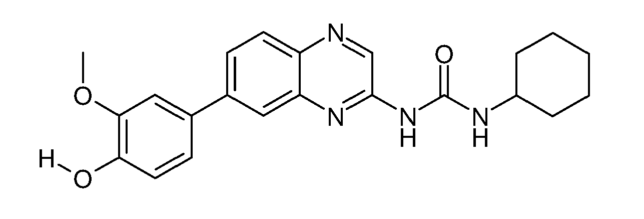

¹HNMR (300MHz, DMSO-*d₆*) δ= 9.99 (1H, s), 8.80 (1H, m), 8.75 (1H, s), 7.95 (3H, s), 7.25 (2H, s), 6.90 (1H, m),3.90 (3H, m), 3.68 (1H, s),1.7 (4H, s),1.4 (6H, s) ppm
MS (ESI) m/z 392 (MH⁺)

### Beispiel 3.3: Cyclohexyl-3-[7-(3,4-dimethoxy-phenyl)-quinoxalin-2-yl]-urea (3)

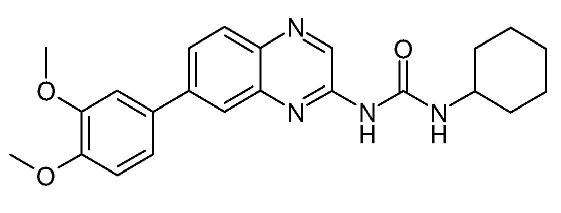

¹HNMR (300MHz, DMSO-*d₆*) δ= 9.86 (1H, m), 8.88 (1H, s), 8.77 (1H, s), 7.95 (3H, s), 7.38 (2H, s), 7.11(1H, m), 3.90 (3H, s) 3.80 (3H, s), 3.36 (1H, s),1.75 (2H, s),1.52 (6H,m) ppm
MS (ESI) m/z 407 (MH⁺)

### Beispiel 3.4: 1-[7-(3,4-Dimethoxy-phenyl)-quinoxalin-2-yl]-3-[1-(2,2,2-trifluoro-acetyl)-piperidin-4-yl]-urea (4)

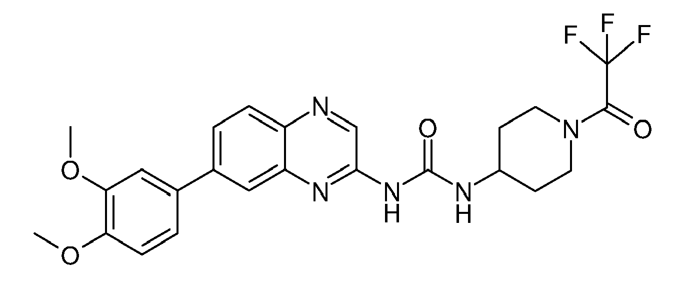

¹HNMR (300MHz, DMSO-*d₆*) δ= 10.13 (1H, s), 8.89 (1H, m), 8.82 (1H, s), 8.07 (1H, m), 7.96 (2H, s), 7.35 (2H, s), 7.00 (1H, s), 4.22 (2H, m), 4.15 (1H, m), 3.90 (3H, s), 3.82 (3H, s), 3.49 (1H, s), 3.25 (1H, s), 2.04 (2H, m), 1.65 (2H, m) ppm
MS (ESI) m/z 504 (MH⁺)

### Beispiel 3.5: 1-[7-(4-Hydroxy-3-methoxy-phenyl)-quinoxalin-2-yl]-3-phenyl-urea (5)

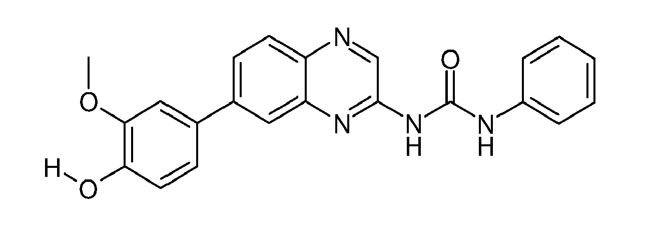

¹HNMR (300MHz, DMSO-*d₆*) δ= 10.98 (1H, s), 10.40 (1H, s), 9.30 (1H, m), 8.88 (1H, s), 8.20 (3H, m), 7.68 (2H, s), 7.52 (4H, s), 6.90 (2H, m), 3.90 (3H, s) ppm
MS (ESI) m/z 386 (MH⁺)

### Beispiel 3.6: 1-[7-(4-Hydroxy-3-methoxy-phenyl)-quinoxalin-2-yl]-3-[1-(2,2,2-trifluoroacetyl)-piperidin-4-yl]-urea (6)

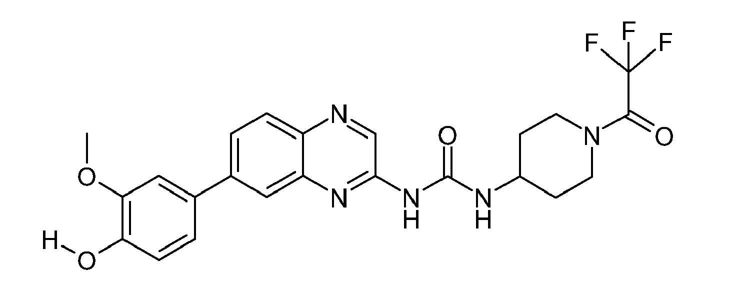

¹HNMR (300MHz, DMSO-*d₆*) δ= 10.12 (1H, s), 9.30 (1H, m), 8.88 (1H, s), 8.80 (1H, m), 8.78 (3H, s), 7.35 (2H, s), 6.90 (1H, m), 4.15 (2H, m), 3.90 (3H, s), 3.33 (2H, m), 2.15 (2H, m), 1.75 (2H, m) ppm
MS (ESI) m/z 489 (MH⁺)

### Beispiel 3.7: 1-Benzyl-3-[7-(4-hydroxy-3-methoxy-phenyl)-quinoxalin-2-yl]-urea (7)

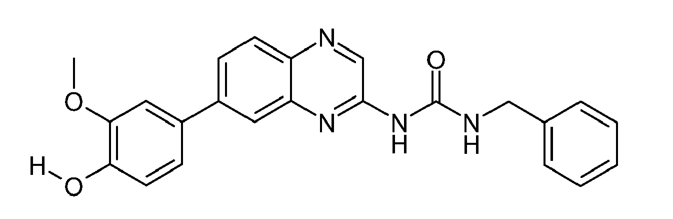

¹HNMR (300MHz, DMSO-*d₆*) δ= 10.50 (1H, s), 9.30 (1H, m), 8.88 (1H, s), 8.00 (3H, s), 7.41 (5H, s), 7.25 (2H, m), 6.90 (1H, m), 4.53 (2H, m), 3.90 (3H, s) ppm
MS (ESI) m/z 400 (MH⁺)

### Beispiel 3.8: 1-Cyclopentyl-3-[7-(3,4-dimethoxy-phenyl)-quinoxalin-2-yl]-urea (8)

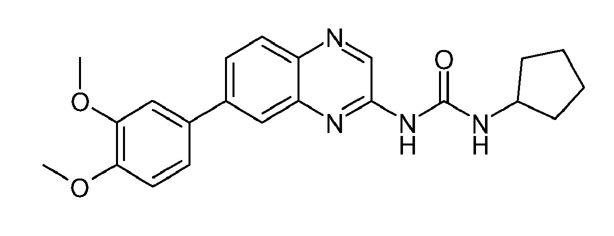

¹HNMR (300MHz, DMSO-*d₆*) δ= 9.86 (1H, m), 8.88 (1H, s), 8.77 (1H, s), 7.95 (3H, s), 7.38 (2H, s), 7.11(1H, m), 4.12 (1H, s), 3.90 (3H, s) 3.80 (3H, s), 1.75 (2H, s),1.52 (6H,m) ppm
MS (ESI) m/z 393 (MH⁺)

### Beispiel 3.9: 1-tert-Butyl-3-[7-(4-hydroxy-3,5-dimethyl-phenyl)-quinoxalin-2-yl]-urea (9)

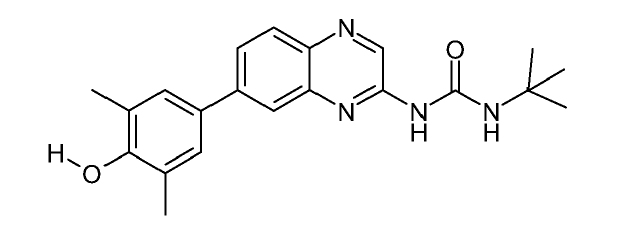

¹HNMR (300MHz, DMSO-*d₆*) δ= 9.84 (1H, s), 9.88 (1H, s), 8.76 (1H, m), 8.58 (1H, s), 7.89 (3H, s), 7.45 (2H, s),2.28 (6H, s),1.40 (9H, m) ppm
MS (ESI) m/z 365 (MH⁺)

### Beispiel 3.10: 1-tert-Butyl-3-[7-(4-hydroxy-3-methoxy-phenyl)-quinoxalin-2-yl]-urea (10)

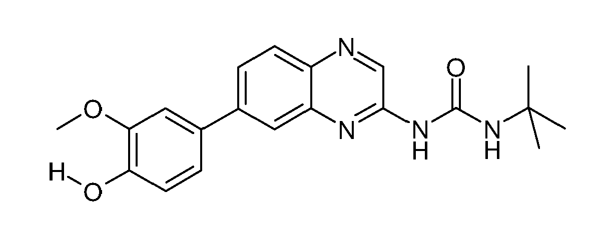

¹HNMR (300MHz, DMSO-*d₆*) δ= 10.50 (1H, s), 9.30 (1H, m), 8.88 (1H, s), 8.20 (1H, m), 8.81 (1H, s), 7.91 (3H, s), 7.32 (2H, s), 6.90 (1H, m), 3.90 (3H, s),1.42 (9H, m) ppm
MS (ESI) m/z 367 (MH⁺)

### Beispiel 3.11: 1-[7-(4-Hydroxy-3-methoxy-phenyl)-quinoxalin-2-yl]-3-thiophen-2-yl-urea (11)

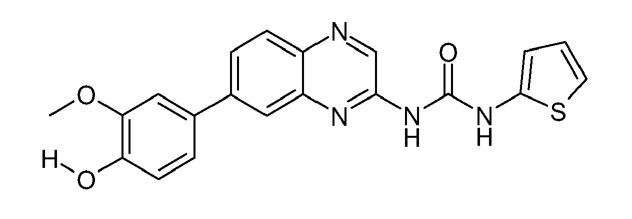

¹HNMR (300MHz, DMSO-*d₆*) δ= 10.50 (1H, s), 9.30 (1H, m), 8.88 (1H, s), 8.20 (1H, m), 8.00 (2H, s), 7.35 (2H, s), 7.00 (2H, s), 6.90 (2H, m), 3.90 (3H, s) ppm
MS (ESI) m/z 393 (MH⁺)

### Beispiel 3.12: 1-Cyclohexyl-3-[7-(4-hydroxy-3,5-dimethyl-phenyl)-quinoxalin-2-yl]-urea (12)

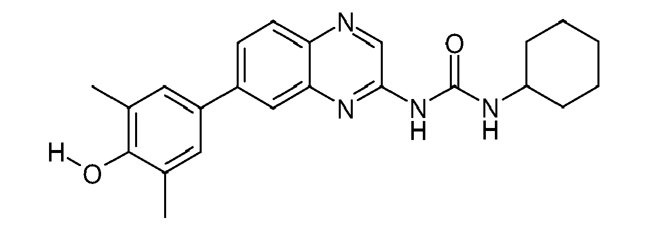

¹HNMR (300MHz, DMSO-*d₆*) δ= 9.98 (1H, s), 8.86 (1H, m), 8.58 (1H, s), 7.98 (1H, m), 7.89 (2H, s), 7.45 (2H, s), 3.34 (1H, s), 2.28 (6H, s),1.88 (2H, s), 1.70 (3H, m), 1.35 (5H, m) ppm
MS (ESI) m/z 391 (MH⁺)

### Beispiel 3.13: 1-Cyclopentyl-3-[7-(4-hydroxy-3,5-dimethyl-phenyl)-quinoxalin-2-yl]-urea (13)

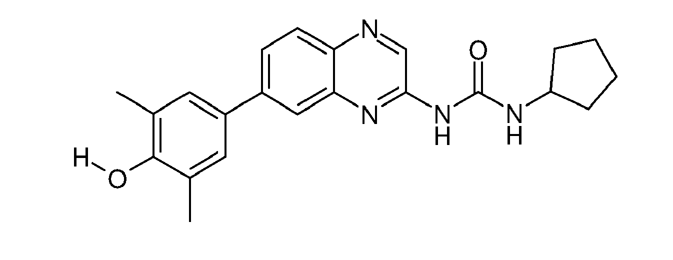

¹HNMR (300MHz, DMSO-*d₆*) δ= 9.98 (1H, s), 8.86 (1H, m), 8.58 (1H, s), 7.98 (1H, m), 7.89 (2H, s), 7.45 (2H, s),4. 11 (1H, s), 2.28 (6H, s), 1.96 (2H, s),1.70 (6H, m) ppm
MS (ESI) m/z 377 (MH⁺)

### Beispiel 3.14: 1-[7-(4-Hydroxy-3,5-dimethyl-phenyl)-quinoxalin-2-yl]-3-phenyl-urea (14)

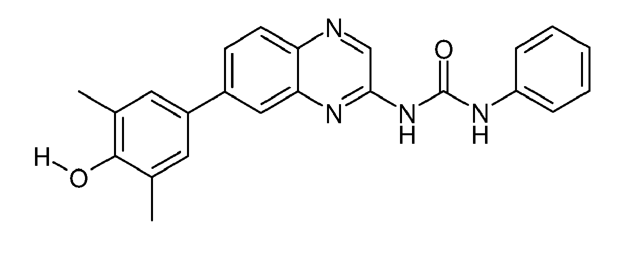

¹HNMR (300MHz, DMSO-*d₆*) δ= 10.98 (1H, s), 8.88 (1H, s), 8.00 (3H, m), 7.86 (2H, s), 7.42 (4H, s), 7.00 (1H, s), 2.25 (6H, m) ppm
MS (ESI) m/z 385 (MH⁺)

### Beispiel 3.15: 1-[7-(4-Hydroxy-3,5-dimethyl-phenyl)-quinoxalin-2-yl]-3-thiophen-2-yl-urea (15)

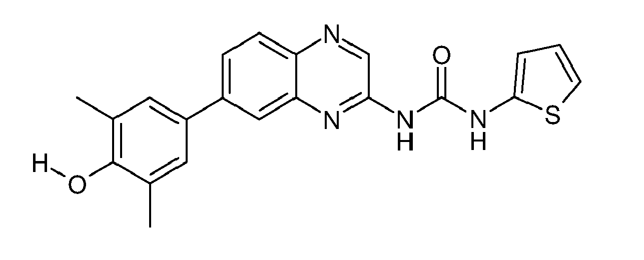

¹HNMR (300MHz, DMSO-*d₆*) δ= 11.85 (1H, s),10.56 (1H, s), 9.98 (1H, s), 8.96 (1H, m), 8.58 (1H, s), 8.22 (1H, s), 7.98 (2H, m), 7.52 (2H, s), 7.00 (2H, s), 6.95 (1H, s), 2.28 (6H, s) ppm
MS (ESI) m/z 391 (MH⁺)

### Beispiel 3.16: 1-[7-(4-Hydroxy-3,5-dimethyl-phenyl)-quinoxalin-2-yl]-3-methyl-urea (16)

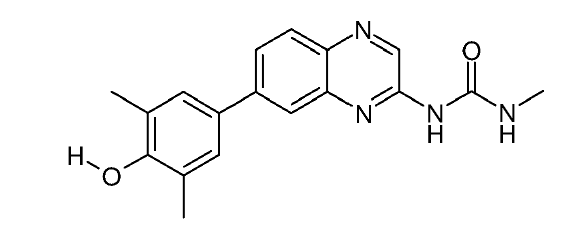

m.p.: 251°C
¹HNMR (600MHz, DMSO-*d₆*) δ= 10.1 (1H, s), 8.82 (1H, m), 8.69 (1H, s), 8.52 (1H, s), 8.08 (1H, s), 7.92 (1H, m), 7.85 (1H, s), 7.44 (2H, s), 2.85 (3H, s), 2.25 (6H, s) ppm
MS (ESI) m/z 323 (MH⁺)

### Beispiel 3.17: 1-[7-(4-Hydroxy-3,5-dimethyl-phenyl)-quinoxalin-2-yl]-3-isopropyl-urea (17)

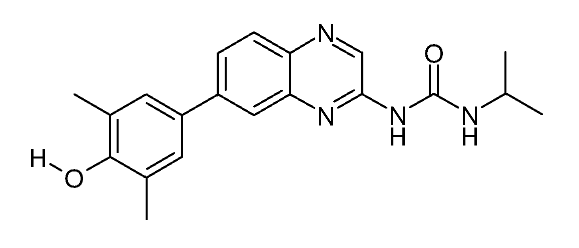

m.p.: 280-283°C
¹HNMR (600MHz, DMSO-*d₆*) δ= 9.92 (1H, s), 8.82 (1H, s), 8.54 (1H, s), 7.95-7.85 (3H, m), 7.42 (2H, s), 3.85 (1H, m), 2.25 (6H, s), 1.22 (6H, d) ppm
MS (ESI) m/z 351 (MH⁺)

### Beispiel 3.18: 1-Butyl-3-[7-(4-hydroxy-3,5-dimethyl-phenyl)-quinoxalin-2-yl]-urea (18)

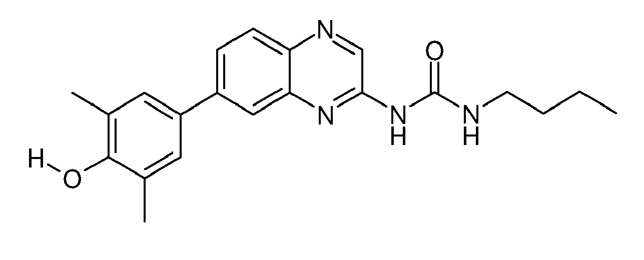

m.p.: 276-280°C
¹HNMR (600MHz, DMSO-*d₆*) δ= 10.02 (1H, s), 8.85 (1H, s), 8.73 (1H, s), 8.53 (1H, s), 7.96-7.83 (3H, m), 7.42 (2H, s), 3.28 (2H, m), 2.48 (6H, s), 1.52 (2H, m), 1.38 (2H, m), 0.95 (3H, m) ppm
MS (ESI) m/z 365 (MH⁺)

### Beispiel 3.19: 1-Ethyl-3-[7-(4-hydroxy-3,5-dimethyl-phenyl)-quinoxalin-2-yl]-urea (19)

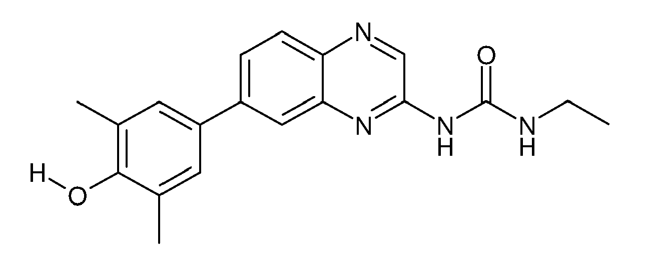

m.p.: 257°C
¹HNMR (600MHz, DMSO-*d₆*) δ= 10.0 (1H, s), 8.82 (1H, m), 8.74 (1H, s), 8.22 (1H, m), 7.94-7.84 (2H, m), 7.43 (2H, s), 3.28 (2H, m), 2.23 (6H, s), 1.18 (3H, m) ppm
MS (ESI) m/z 337 (MH⁺)

### Beispiel 3.20: 1-Cyclohexyl-3-[7-(4-hydroxy-3,5-dimethyl-phenyl)-quinoxalin-2-yl]-thiourea (20)

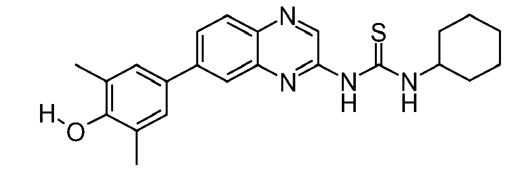

¹HNMR (600MHz, DMSO-*d₆*) δ= 11.78 (1H, s), 11.16 (1H, s), 8.75 (1H, s), 8.55 (1H, m), 7.96-7.85 (3H, m), 7.41 (2H, s), 4.35 (1H, m), 3.28 (2H, m), 2.28 (6H, s), 1.95 (2H, m),1.77 (2H, m), 1.62 (3H, m), 1.45 (3H, m) ppm
MS (ESI) m/z 407 (MH⁺)

### Beispiel 3.21: 1-Cyclohexyl-3-[7-(3,5-dichloro-4-hydroxy-phenyl)-quinoxalin-2-yl]-thiourea (21)

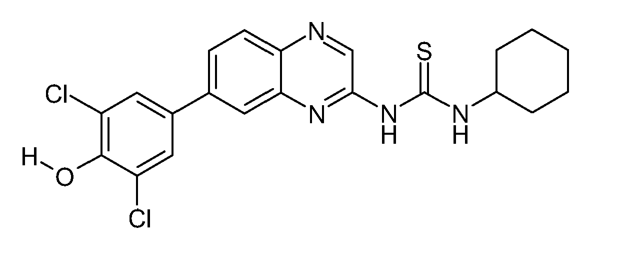

m.p.: 284°C
¹HNMR (600MHz, DMSO-*d₆*) δ= 11.64 (1H, s), 11.2 (1H, s), 10.48 (1H, s), 8.79 (1H, s), 8.03-7.96 (3H, m), 7.87 (2H, s), 4.22 (1H, m), 2.02 (2H, m),1.85 (2H, m), 1.62 (3H, m), 1.4 (3H, m) ppm

### Beispiel 3.22: 1-Ethyl-3-[7-(4-hydroxy-3,5-dimethyl-phenyl)-quinoxalin-2-yl]-thiourea (22)

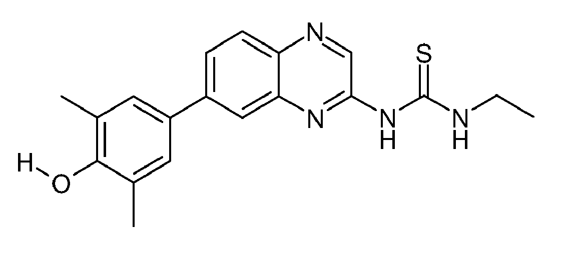

m.p.: 243-244°C
¹HNMR (600MHz, DMSO-*d₆*) δ= 11.65 (1H, s), 11.14 (1H, s), 8.74 (1H, s), 8.55 (1H, m), 8.15 (1H, m), 7.97-7.7.91 (2H, m), 7.41 (2H, s), 3.78 (2H, m), 2.24 (6H, s), 1.28 (3H, m) ppm
MS (ESI) m/z 353 (MH⁺)

### Beispiel 3.23: 1-Cyclopropyl-3-[7-(4-hydroxy-3,5-dimethyl-phenyl)-quinoxalin-2-yl]-thiourea (23)

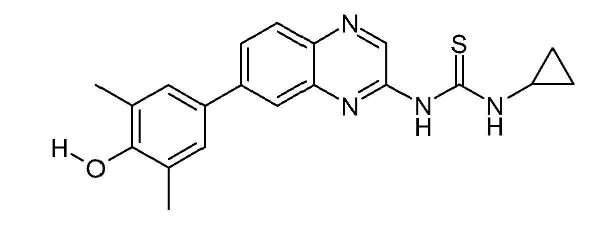

m.p.: 274-276°C
¹HNMR (600MHz, DMSO-*d₆*) δ= 11.61 (1H, s), 11.23 (1H, s), 8.73 (1H, s), 8.53 (1H, m), 7.99-7.7.91 (3H, m), 7.46 (2H, s), 3.22 (1H, m), 0.91-0.78 (4H, m) ppm
MS (ESI) m/z 365 (MH⁺)

### Beispiel 3.24: 1-Cyclopentyl-3-[7-(4-hydroxy-3,5-dimethyl-phenyl)-quinoxalin-2-yl]-thiourea (24)

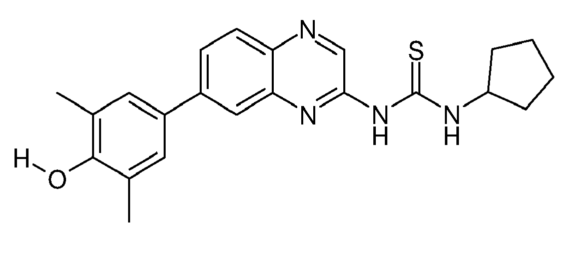

m.p.: 271-272°C
MS (ESI) m/z 393 (MH⁺)

### Beispiel 3.25: 1-tert-Butyl-3-[7-(4-hydroxy-3,5-dimethyl-phenyl)-quinoxalin-2-yl]-thiourea (25)

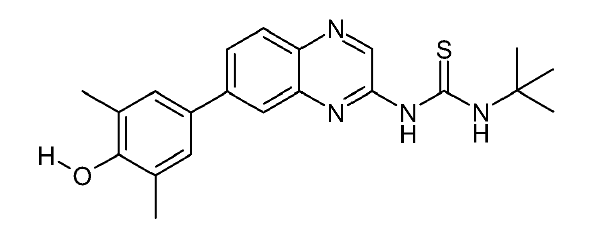

m.p.: 238-240°C
MS (ESI) m/z 381 (MH⁺)

### Beispiel 3.26: 1-Dodecyl-3-(7-phenyl-quinoxalin-2-yl)-urea (26)

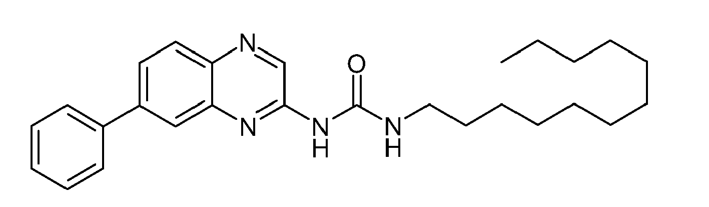

¹HNMR (300MHz, DMSO-*d₆*) δ= 10.14 (1H, s), 8.93 (1H, m), 8.81 (1H, s), 8.00 (5H, m), 7.45 (3H, s), 3.31 (2H, s), 1.57 (2H, s), 1.24 (20H, m), 0.83 (3H, s) ppm
MS (ESI) m/z 433 (MH⁺)

### Beispiel 3.27: 1-(3-Chloro-2-methyl-phenyl)-3-(7-phenyl-quinoxalin-2-yl)-urea (27)

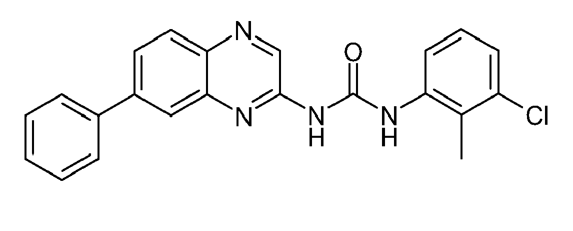

¹HNMR (300MHz, DMSO-*d₆*) δ= 10.77 (1H, s), 10.14 (1H, s), 8.93 (1H, m), 8.00 (5H, m), 7.45 (5H, s), 7.22 (1H, s), 2.21 (3H, s) ppm
MS (ESI) m/z 389 (MH⁺)

### Beispiel 3.28: 1-(3,4-Dimethyl-phenyl)-3-(7-phenyl-quinoxalin-2-yl)-urea (28)

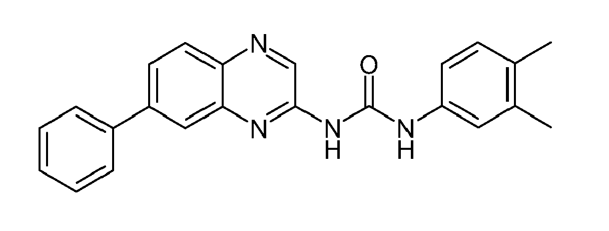

¹HNMR (300MHz, DMSO-*d₆*) δ= 10.77 (1H, s), 10.14 (1H, s), 8.93 (1H, m), 8.00 (5H, m), 7.45 (5H, s), 7.22 (1H, s), 2.21 (6H, s) ppm
MS (ESI) m/z 369 (MH⁺)

### Beispiel 3.29: 1-Allyl-3-(7-phenyl-quinoxalin-2-yl)-urea (29)

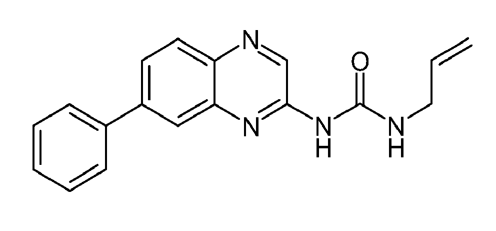

¹HNMR (300MHz, DMSO-*d₆*) δ= 10.14 (1H, s), 8.88 (2H, m), 8.00 (5H, m), 7.45 (3H, s), 6.00 (1H, s), 5.21 (2H, s), 3.95 (2H, m) ppm
MS (ESI) m/z 305 (MH⁺)

### Beispiel 3.30: 1-Cyclopentyl-3-(7-phenyl-quinoxalin-2-yl)-urea (30)

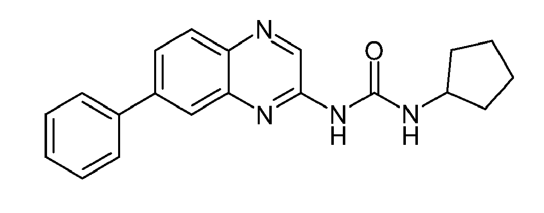

¹HNMR (300MHz, DMSO-*d₆*) δ= 10.14 (1H, s), 8.93 (2H, m), 8.00 (5H, m), 7.45 (3H, s), 3.61 (1H, s), 1.57 (8H, s) ppm
MS (ESI) m/z 333 (MH⁺)

### Beispiel 3.31: 1-[7-(4-Hydroxy-3,5-dimethyl-phenyl)-quinoxalin-2-yl]-3-isopropyl-thiourea (31)

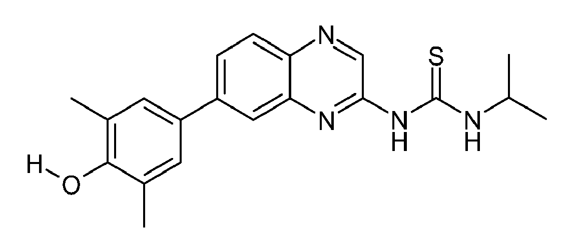

m.p.: 269-271 °C
MS (ESI) m/z 367 (MH⁺)

### Beispiel 3.32: 1-Butyl-3-[7-(4-hydroxy-3,5-dimethyl-phenyl)-quinoxalin-2-yl]-thiourea (32)

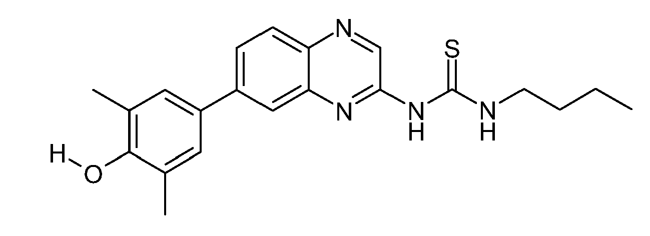

m.p.: 278-280°C
MS (ESI) m/z 381 (MH⁺)

### Beispiel 3.33: 1-[7-(4-Hydroxy-3,5-dimethyl-phenyl)-quinoxalin-2-yl]-3-pentyl-urea (33)

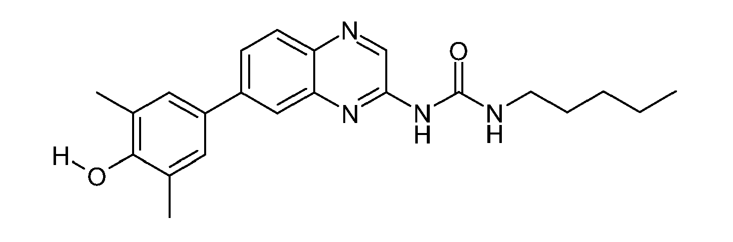

m.p.: 274-275°C
MS (ESI) m/z 379 (MH⁺)

### Beispiel 3.34: 1-Cyclobutyl-3-[7-(4-hydroxy-3,5-dimethyl-phenyl)-quinoxalin-2-yl]-thiourea (34)

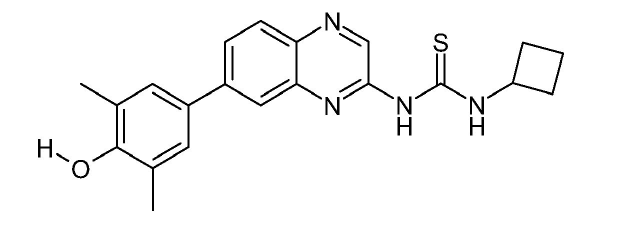

m.p.: 285-286°C
MS (ESI) m/z 379 (MH⁺)

### Beispiel 3.35: 1-Hexyl-3-[7-(4-hydroxy-3,5-dimethyl-phenyl)-quinoxalin-2-yl]-urea (35)

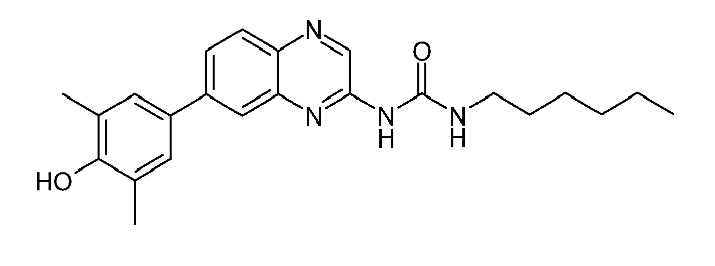

m.p.: 272-273°C
MS (ESI) m/z 393 (MH⁺)

### Beispiel 3.36: 1-[7-(4-Hydroxy-3,5-dimethyl-phenyl)-quinoxalin-2-yl]-3-propyl-urea (36)

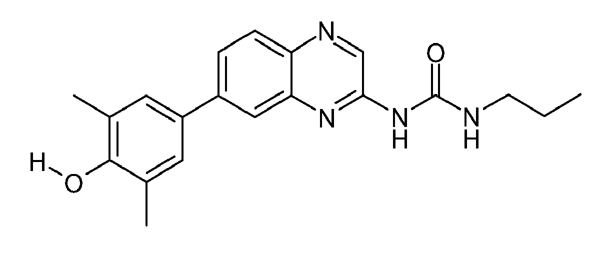

m.p.: 276-277°C
MS (ESI) m/z 351 (MH⁺)

### Beispiel 3.37: 1-Dodecyl-3-[7-(4-hydroxy-3,5-dimethyl-phenyl)-quinoxalin-2-yl]-urea (37)

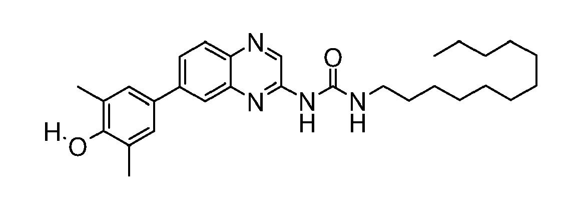

¹HNMR (300MHz, DMSO-*d₆*) δ= 10.14 (1H, s), 8.93 (1H, m), 8.71 (1H, s), 8.51 (1H, s),7.95 (3H, m), 7.45 (2H, s), 2.26 (6H, s), 1.57 (2H, s), 1.24 (20H, m) ppm
MS (ESI) m/z 477 (MH⁺)

### Beispiel 3.38: 1-(3-Chloro-2-methyl-phenyl)-3-[7-(4-hydroxy-3,5-dimethyl-phenyl)-quinoxalin-2-yl]-urea (38)

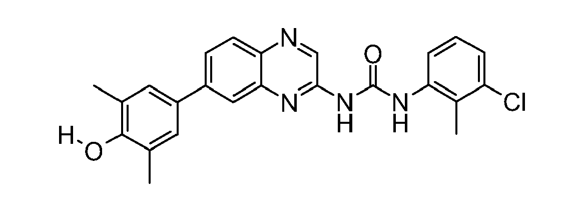

¹HNMR (300MHz, DMSO-*d₆*) δ= 11.14 (1H, s),10.14 (1H, s), 8.93 (1H, m), 8.51 (1H, s),7.95 (4H, m), 7.45 (4H, s), 7.15 (1H, s),2.26 (6H, s), 2.15 (3H, s) ppm
MS (ESI) m/z 433 (MH⁺)

### Beispiel 3.39: 1-(3-Acetyl-phenyl)-3-[7-(4-hydroxy-3,5-dimethyl-phenyl)-quinoxalin-2-yl]-urea (39)

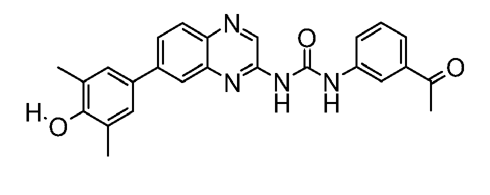

¹HNMR (300MHz, DMSO-*d₆*) δ= 11.14 (1H, s),10.14 (1H, s), 8.93 (1H, m), 8.51 (1H, s),7.95 (4H, m), 7.45 (2H, s), 7.15 (2H, s), 2.56 (3H, s), 2.15 (3H, s) ppm
MS (ESI) m/z 427 (MH⁺)

### Beispiel 3.40: 1-(3,4-Dimethyl-phenyl)-3-[7-(4-hydroxy-3,5-dimethyl-phenyl)-quinoxalin-2-yl]-urea (40)

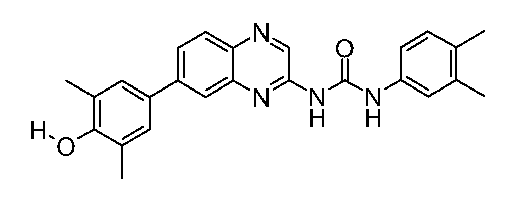

¹HNMR (300MHz, DMSO-*d₆*) δ= 11.14 (1H, s),10.14 (1H, s), 8.93 (1H, m), 8.51 (1H, s),7.95 (4H, m), 7.45 (4H, s), 7.15 (1H, s), 2.26 (6H, s), 2.15 (6H, s) ppm
MS (ESI) m/z 413 (MH⁺)

### Beispiel 3.41: 1-Allyl-3-[7-(4-hydroxy-3,5-dimethyl-phenyl)-quinoxalin-2-yl]-urea (41)

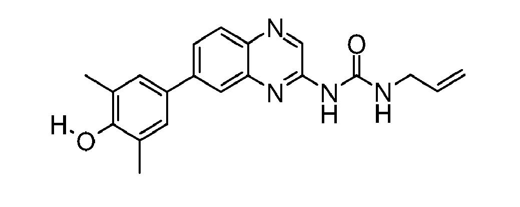

¹HNMR (300MHz, DMSO-*d₆*) δ= 10.14 (1H, s), 8.93 (1H, m), 8.71 (1H, s), 8.54 (1H, s), 7.45 (3H, s), 5.97 (1H, s), 5.22 (2H, s), 3.95 (2H, m), 1.99 (6H, s) ppm
MS (ESI) m/z 349 (MH⁺)

### Beispiel 3.42: 1-Cyclooctyl-3-[7-(4-hydroxy-3,5-dimethyl-phenyl)-quinoxalin-2-yl]-urea (42)

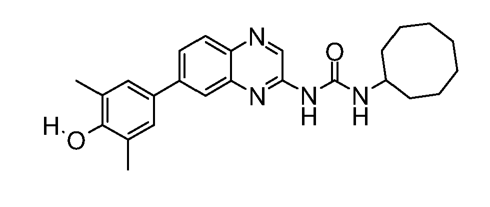

¹HNMR (300MHz, DMSO-*d₆*) δ=10.14 (1H, s), 8.93 (2H, m), 8.81 (1H, s), 7.85 (3H, m), 7.45 (2H, s), 4.08 (1H, m), 2.28 (6H, m), 1.95 (12H, m), 1.85 (2H, s) ppm
MS (ESI) m/z 419 (MH⁺)

### Beispiel 3.43:1-(2,4-Dimethyl-phenyl)-3-[7-(4-hydroxy-3,5-dimethyl-phenyl)-quinoxalin-2-yl]-urea (43)

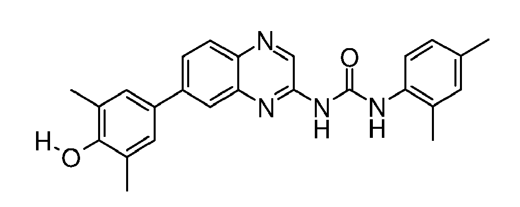

¹HNMR (300MHz, DMSO-*d₆*) δ= 11.14 (1H, s), 10.14 (1H, s), 8.93 (1H, m), 8.51 (1H, s), 7.95 (4H, m), 7.45 (4H, s), 7.15 (1H, s), 2.26 (6H, s), 2.15 (6H, s) ppm
MS (ESI) m/z 413 (MH⁺)

### Beispiel 3.44: 1-Cyclooctyl-3-[7-(4-hydroxy-3-methoxy-phenyl)-quinoxalin-2-yl]-urea (44)

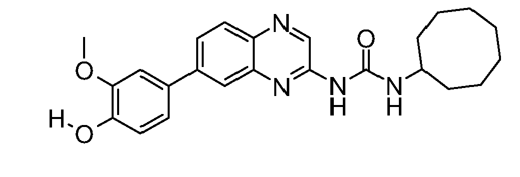

¹HNMR (300MHz, DMSO-*d₆*) δ= 10.14 (1H, s), 9.03 (1H, m), 8.81 (2H, s), 8.00 (3H, m), 7.45 (2H, s), 6.93 (1H, s), 4.08 (1H, s), 3.88 (3H, s), 2.00 (2H, s),1.75 (12H, s) ppm
MS (ESI) m/z 421 (MH⁺)

### Beispiel 3.45: 1-Adamantan-1-yl-3-(7-phenyl-quinoxalin-2-yl)-urea (45)

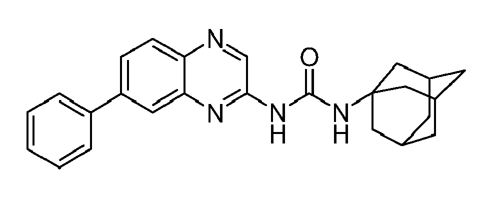

¹HNMR (300MHz, DMSO-*d₆*) δ= 10.14 (1H, s), 8.93 (2H, m), 8.00 (5H, m), 7.45 (3H, s), 2.00 (12H, s), 1.68 (3H, s) ppm
MS (ESI) m/z 399 (MH⁺)

### Beispiel 3.46: 1-[7-(4-Hydroxy-3,5-dimethyl-phenyl)-quinoxalin-2-yl]-3-p-tolyl-urea (46)

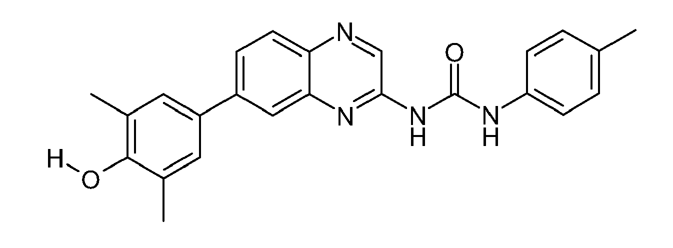

m.p.: >300°C
MS (ESI) m/z 399 (MH⁺)

### Beispiel 3.47: 1-(3,4-Dichloro-phenyl)-3-[7-(4-hydroxy-3,5-dimethyl-phenyl)-quinoxalin-2-yl]-urea (47)

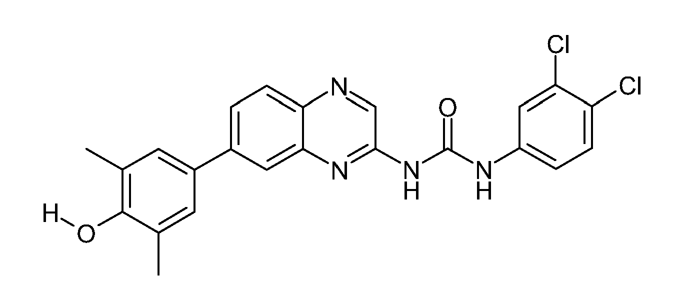

m.p.: >300°C
MS (ESI) m/z 454(MH⁺)

### Beispiel 3.48: 1-[7-(4-Hydroxy-3,5-dimethyl-phenyl)-quinoxalin-2-yl]-3-(4-methoxyphenyl)-urea (48)

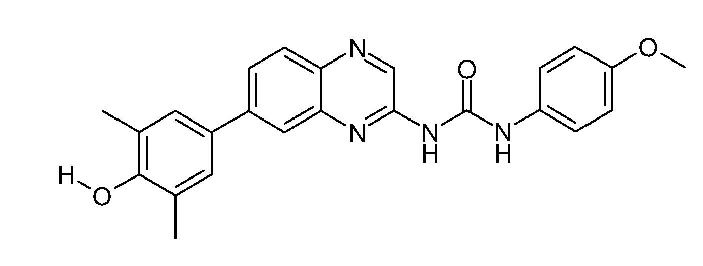

m.p.: >300°C
MS (ESI) m/z 415 (MH⁺)

### Beispiel 3.49: 1-Adamantan-1-yl-3-[7-(4-hydroxy-3,5-dimethyl-phenyl)-quinoxalin-2-yl]-urea (49)

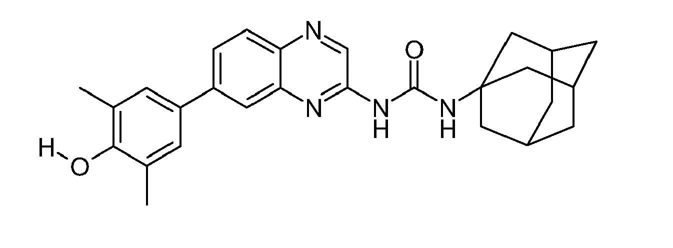

¹HNMR (300MHz, DMSO-*d₆*) δ= 10.14 (1H, s), 8.93 (2H, m), 8.81 (1H, s), 7.85 (3H, m), 7.45 (2H, s), 2.28 (6H, m), 1.85 (12H, m), 1.56 (3H, s) ppm
MS (ESI) m/z 443 (MH⁺)

### Beispiel 3.50: 1-[7-(4-Hydroxy-3,5-dimethyl-phenyl)-quinoxalin-2-yl]-3-naphthalen-2-yl-urea (50)

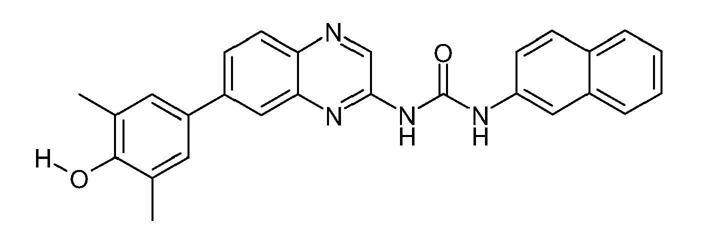

m.p.: >300°C
MS (ESI) m/z 435 (MH⁺)

### Beispiel 3.51: 1-[7-(4-Hydroxy-3,5-dimethyl-phenyl)-quinoxalin-2-yl]-3-(1,1,3,3-tetramethyl-butyl)-urea (51)

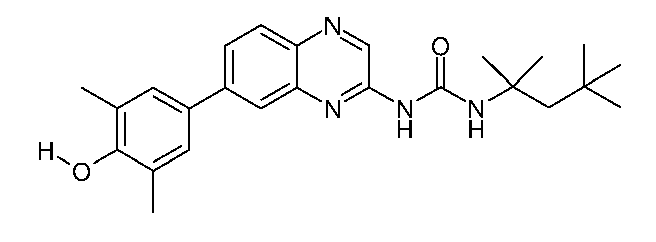

m.p.: >300°C
MS (ESI) m/z 421 (MH⁺)

### Beispiel 3.52: 1-[7-(4-Hydroxy-3,5-dimethyl-phenyl)-quinoxalin-2-yl]-3-pyridin-3-yl-urea (52)

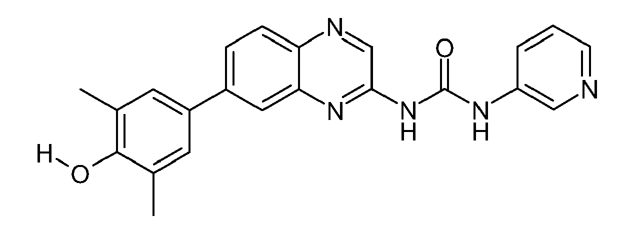

m.p.: 298-300°C
MS (ESI) m/z 386 (MH⁺)

### Beispiel 3.53: 1-((R)-1,2-Dimethyl-propyl)-3-[7-(4-hydroxy-3,5-dimethyl-phenyl)-quinoxalin-2-yl]-urea (53)

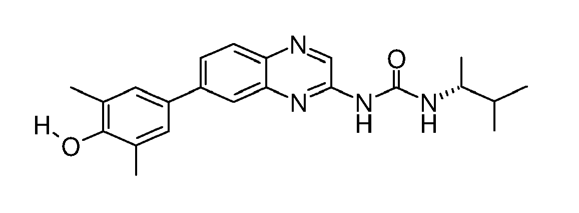

m.p.: 265°C
MS (ESI) m/z 379 (MH⁺)

### Beispiel 3.54: 1-((S)-1,2-Dimethyl-propyl)-3-[7-(4-hydroxy-3,5-dimethyl-phenyl)-quinoxalin-2-yl]-urea (54)

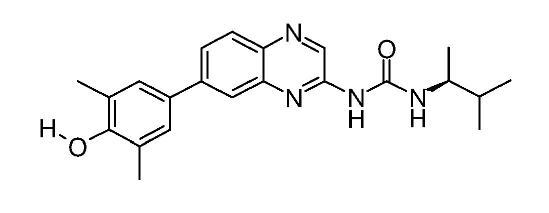

m.p.: 267°C
MS (ESI) m/z 379 (MH⁺)

### Beispiel 3.55: 1-(5-Chloro-2-methyl-phenyl)-3-[7-(4-hydroxy-3,5-dimethyl-phenyl)-quinoxalin-2-yl]-urea (55)

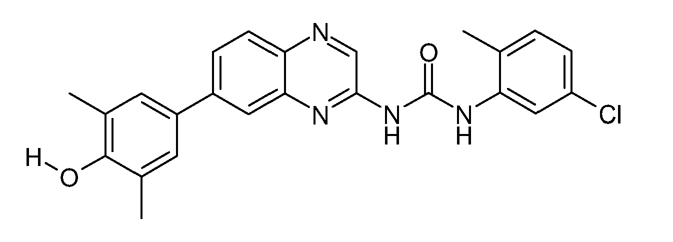

¹HNMR (300MHz, DMSO-*d₆*) δ= 11.14 (1H, s), 10.14 (1H, s), 8.93 (1H, m), 8.51 (1H, s), 7.95 (4H, m), 7.45 (4H, s), 7.15 (1H, s), 2.26 (6H, s), 2.15 (3H, s) ppm
MS (ESI) m/z 433 (MH⁺)

### Beispiel 3.56: 1-((S)-2-Phenyl-cyclopropyl)-3-(7-phenyl-quinoxalin-2-yl)-urea (56)

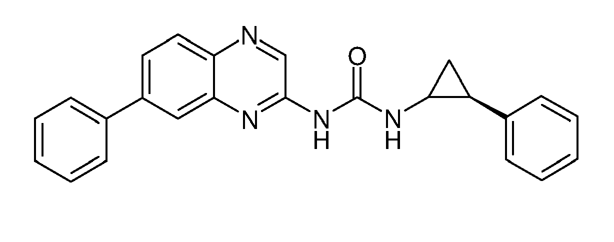

¹HNMR (300MHz, DMSO-*d₆*) δ= 10.14 (1H, s), 8.93 (1H, m), 8.81 (1H, s), 8.00 (5H, m), 7.45 (3H, s), 7.32 (5H, s), 2.95 (1H, s), 2.05 (2H, m), 0.83 (1H, s) ppm
MS (ESI) m/z 381 (MH⁺)

### Beispiel 3.57: 1-[7-(4-Hydroxy-3,5-dimethyl-phenyl)-quinoxalin-2-yl]-3-((S)-2-phenyl-cyclopropyl)-urea (57)

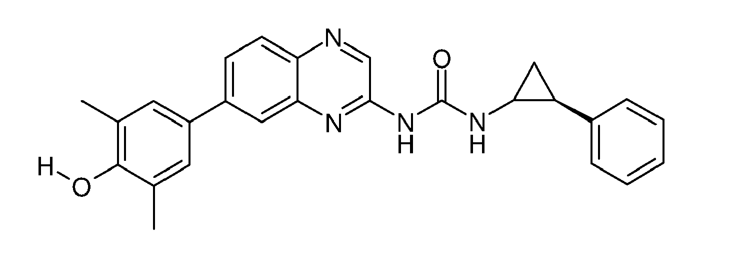

¹HNMR (300MHz, DMSO-*d₆*) δ= 10.14 (1H, s), 8.93 (2H, m), 8.81 (1H, s), 8.00 (3H, m), 7.45 (2H, s), 7.28 (5H, s), 2.93 (1H, s), 2.28 (6H, m), 1.46 (2H, m), 0.83 (1H, s) ppm
MS (ESI) m/z 425 (MH⁺)

### Beispiel 3.58: 1-(2-Chloro-6-methyl-phenyl)-3-[7-(4-hydroxy-3,5-dimethyl-phenyl)-quinoxalin-2-yl]-urea (58)

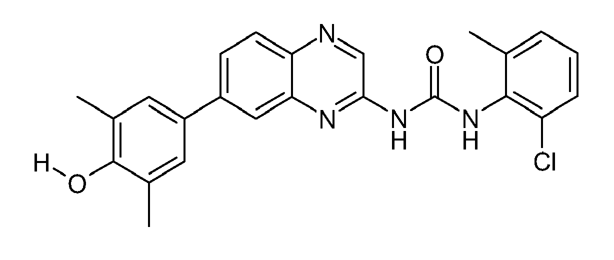

¹HNMR (300MHz, DMSO-*d₆*) δ= 11.14 (1H, s),10.14 (1H, s), 8.93 (1H, m), 8.51 (1H,s), 7.95 (4H, m), 7.45 (4H, s), 7.15 (1H, s), 2.26 (6H, s), 2.15 (3H, s) ppm
MS (ESI) m/z 433 (MH⁺)

### Beispiel 3.59: 1-Allyl-3-[7-(4-hydroxy-3-methoxy-phenyl)-quinoxalin-2-yl]-urea (59)

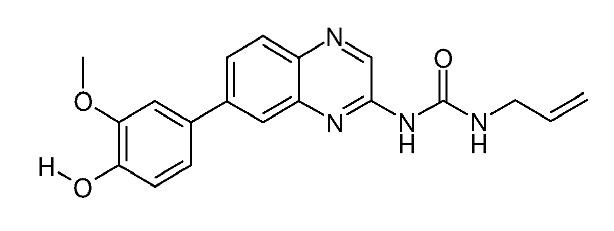

¹HNMR (300MHz, DMSO-*d₆*) δ= 10.14 (1H, s), 9.03 (1H, m), 8.81 (2H, s), 8.00 (3H, m), 7.45 (2H, s), 6.93 (1H, s), 5.96 (1H, s), 5.21 (2H, m), 3.94 (2H, s), 3.88 (3H, s) ppm
MS (ESI) m/z 351 (MH⁺)

### Beispiel 3.60: 1-(3,5-Dimethyl-isoxazol-4-yl)-3-[7-(4-hydroxy-3,5-dimethyl-phenyl)-quinoxalin-2-yl]-urea (60)

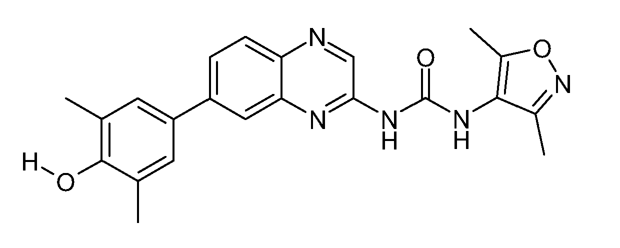

¹HNMR (300MHz, DMSO-*d₆*) δ= 12.48 (1H, s), 8.42 (1H, s), 8.05 (1H, s), 7.84 (2H, s), 7.41 (1H, s), 2.47 (3H, s), 2.24 (6H, s), 2.18 (3H, s) ppm
MS (ESI) m/z 404 (MH⁺)

### Beispiel 3.61: 1-sec-Butyl-3-[7-(4-hydroxy-3,5-dimethyl-phenyl)-quinoxalin-2-yl]-urea (61)

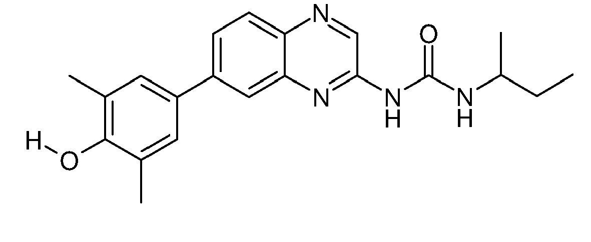

¹HNMR (300MHz, DMSO-*d₆*) δ= 9.85 (1H, s), 8.84 (1H, s), 8.53-8.51 (2H, m), 7.93 (1H, d), 7.84 (2H, m), 7.41 (2H, s), 3.76 (1H, m), 2.24 (6H, s), 1.54 (2H, m), 1.21 (3H, d), 0.88 (3H, t) ppm
MS (ESI) m/z 365 (MH⁺)

### Beispiel 3.62: 1-Ethyl-3-[7-(4-methoxy-3,5-dimethyl-phenyl)-quinoxalin-2-yl]-urea (62)

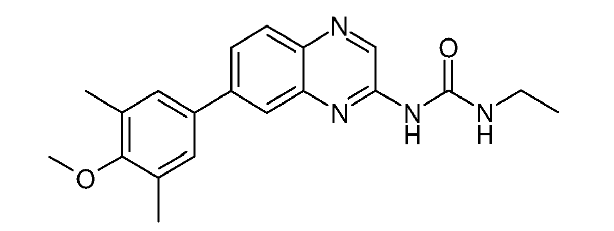

¹HNMR (300MHz, DMSO-*d₆*) δ= 10.03 (1H, s), 8.79 (2H, s), 8.08 (1H, d), 7.96 (1H, d), 7.88 (1H, m), 7.52 (2H, s), 3.71 (3H, s), 3.31 (2H, s), 2.33 (6H, s), 1.19 (3H, t) ppm MS (ESI) m/z 351 (MH⁺)

### Beispiel 3.63: 1-Isopropyl-3-[7-(4-methoxy-3,5-dimethyl-phenyl)-quinoxalin-2-yl]-urea (63)

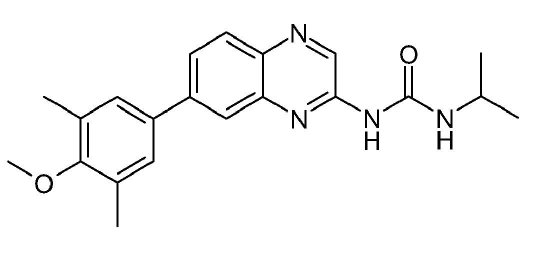

¹HNMR (300MHz, DMSO-*d₆*) δ= 9.94 (1H, s), 8.87 (1H, s), 8.53-8.52 (1H, d), 7.98-7.95 (2H, m), 7.88-7.86 (2H, m), 7.51 (2H, s), 3.94-3.91(1H, m), 3.71 (3H, s), 2.33 (6H, s), 1.25-1.24(6H, d) ppm
MS (ESI) m/z 365 (MH⁺)

### Beispiel 3.64: 1-Isopropyl-3-[7-(4-methoxy-3,5-dimethyl-phenyl)-quinoxalin-2-yl]-thiourea (64)

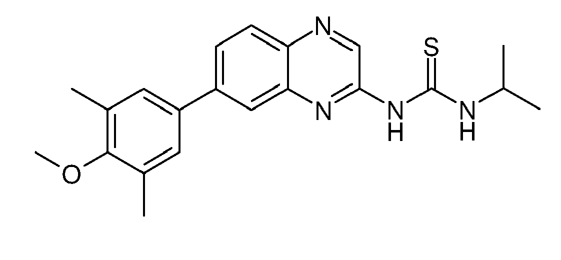

¹HNMR (300MHz, DMSO-*d₆*) δ= 11.63 (1H, d), 11.17 (1H, s), 8.77 (1H, s), 7.99 (2H, m), 7.92 (1H, m), 7.52 (2H, s), 4.49-4.46 (1H, m), 3.71 (3H, s), 2.34 (6H, s), 1.38 (6H, d) ppm
MS (ESI) m/z 381 (MH⁺)

### Beispiel 3.65: 1-[7-(4-Hydroxy-3,5-dimethyl-phenyl)-quinoxalin-2-yl]-3-isobutyl-urea (65)

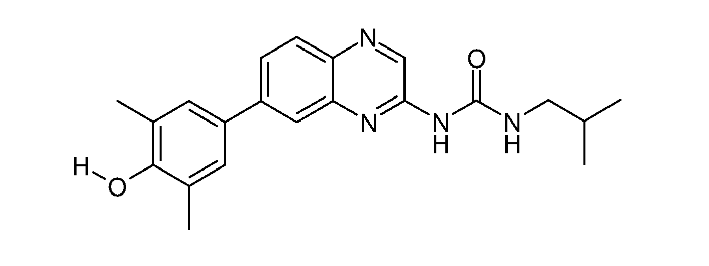

¹HNMR (300MHz, DMSO-*d*₆) δ= 10.02 (1H, s), 8.85 (1H, m), 8.79 (1H, s), 8.53 (1H, s), 7.94-7.85 (3H, m), 7.41 (2H, s), 3.15-3.13 (2H, t), 2.27 (6H, s), 1.88-1.84 (1H, m), 0.95 (6H, d) ppm
MS (ESI) m/z 365 (MH⁺)

### Beispiel 3.66: 1-(1-Ethyl-propyl)-3-[7-(4-hydroxy-3,5-dimethyl-phenyl)-quinoxalin-2-yl]-urea (66)

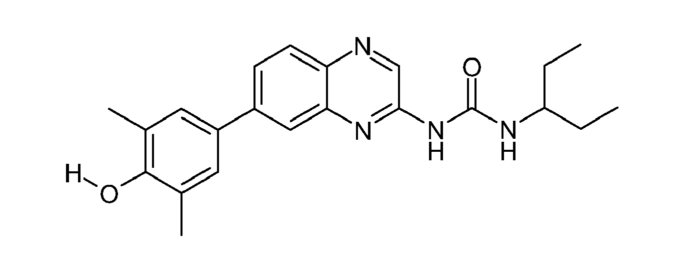

¹HNMR (300MHz, DMSO-*d₆*) δ= 9.92 (1H, s), 8.85 (1H, s), 8.52 (2H, s), 7.95 (1H, d), 7.85 (2H, m), 7.40 (2H, s), 3.67-3.64 (1H, m), 2.27 (6H, s), 1.63-1.52 (4H, m), 0.94-0.92 (6H, t) ppm
MS (ESI) m/z 379 (MH⁺)

### Biologische Wirkungen der erfindungsgemäßen Verbindungen

### Beispiel 4 Antiproliferative Wirkung an verschiedenen Tumorzelllinien

Die antiproliferative Wirkung der erfinderischen Verbindungen der allgemeinen Formel I wurde gemäß den Proliferationstests:
a) Alamar Blue-Assay (Page et al. Int. J. Oncology 1993, 3, 473)
b) XTT-Assay (Scuderio et al. Cancer Res. 1988, 48, 4827)
bestimmt.

Bei den verwendeten Zelllinien handelt es sich um die Zelllinie KB/HeLa (Zervix), die Zelllinie SKOV-3 (Ovar), die Zelllinie NCI-H460 (Lunge), die Zelllinie PC3 (Prostata), die HCT-116 (Kolon) , die Zelllinie MDA-MB468 (Brust), die Zelllinie A549 (Lunge), die Zelllinie U87MG (Gliom), die Zelllinie L363 (Leukämie), die Zelllinie TMM (Leukämie), die Zelllinie RKOp27 (Kolon), die Zelllinie RS4,11 (Leukämie), die Zelllinie HSB-2 (Leukämie), die Zelllinie NALM-6 (Leukämie), die Zelllinie MOLT-3 (Leukämie), die Zelllinie MOLT-16 (Leukämie), die Zelllinie MCF-7 (Brust), die Zelllinie MDA-MBA435 (Brust) und die Zelllinie LnCap (Prostata).

In den Tabellen 1a und 1b sind die zytotoxischen bzw. wachstumshemmenden Aktivitäten der erfinderischen Verbindungen gezeigt. Die Ergebnisse zeigen eine sehr potente Hemmung der Proliferation ausgewählter Tumorzelllinien durch die genannten Substanzen.

**Tabelle 1a**

| | Proliferationsassay, EC50 in [µM] | | | | | | | | | |
|---|---|---|---|---|---|---|---|---|---|---|
| **Vbb** | **NCI-H460** | **SKOV-3** | **HCT 116** | **MDA-MB468** | **PC-3** | **A549** | **U87MG** | **L363** | **TMM** | **RKOP27** |
| **17** | 0.047 | 0.04 | 0.024 | 0.039 | 0.028 | 0.029 | 0.010 | 0.078 | 0.032 | 0.026 |
| **9** | 0.238 | 0.248 | 0.229 | 0.263 | 0.112 | 0.289 | 0.181 | n.b. | n.b. | 0.176 |
| **12** | 0.085 | 0.067 | 0.042 | 0.125 | 0.051 | 0.076 | 0.077 | n.b. | n.b. | n.b. |
| **15** | 0.714 | 1.81 | 0.475 | 0.835 | 0.309 | 0.836 | 1.336 | n.b. | n.b. | n.b. |
| **18** | 0.038 | 0.032 | 0.021 | 0.030 | 0.016 | 0.046 | 0.008 | n.b. | n.b. | n.b. |
| **22** | 0.027 | 0.030 | 0.007 | 0.053 | 0.011 | 0.008 | 0.002 | n.b. | n.b. | 0.028 |
| **23** | 0.021 | 0.016 | 0.003 | 0.148 | 0.003 | 0.009 | 0.002 | n.b. | n.b. | 0.020 |
| **31** | 0.060 | 0.045 | 0.030 | 0.025 | 0.015 | 0.023 | 0.013 | n.b. | n.b. | n.b. |
| **32** | 0.030 | 0.027 | 0.016 | 0.020 | 0.009 | 0.009 | 0.003 | n.b. | n.b. | n.b. |
| **34** | 0.052 | 0.050 | 0.036 | 0.030 | 0.012 | 0.016 | 0.224 | n.b. | n.b. | 0.058 |
| **36** | 0.064 | 0.042 | 0.043 | 0.043 | 0.009 | 0.034 | 0.111 | n.b. | n.b. | n.b. |
| **41** | 0.218 | n.b. | 0.091 | 0.131 | 0.076 | 0.217 | 0.105 | n.b. | n.b. | n.b. |

**Tabelle 1b**

| | Proliferationsassay, EC50 in [µM] | | | | | | | | |
|---|---|---|---|---|---|---|---|---|---|
| **Vbb** | **RS4,11** | **HSB-2** | **NALM-6** | **MOLT-3** | **MOLT-16** | **KB-HeLa** | **MCF-7** | **MDA-MB435** | **LNCaP** |
| **17** | 0.037 | 0.037 | 0.054 | 0.036 | 0.056 | 0.037 | 0.032 | 0.018 | 0.040 |

### Tabelle 1a/ 1 b: Wachstumshemmung erfindungsgemäßer Substanzen im Proliferationsassay an Tumorzelllinien

### Beispiel 5 Antiproliferative Wirkung an resistenten Tumorzelllinien

Zur weiteren Charakterisierung wurden die erfindungsgemäßen Substanzen gegenüber resistenten Tumorzelllinien im Vergleich zu den nicht-resistenten Wildtypzelllinien untersucht.

Bei den untersuchten Zelllinien handelt es sich um die Zelllinie A2780 (Ovar) , um die cis-Platin resistente Zelllinie A2780 (Ovar) um die Zelllinie L1210 (Leukämie), um die Vincristin-resistente Zelllinie L1210 (Leukämie), um die Zelllinie MESSA (Uterus), um die Doxorubicin-resistente Zelllinie MESSA Dx5 (Uterus), um die Zelllinie NCIH69 (Lunge) und um die multi drug resistente Zelllinie NCIH69 AR (Lunge).

In der nachfolgenden Tabelle 3 sind die Ergebnisse zusammengefaßt dargestellt:

**Tabelle 3: Hemmwirkung von Chinoxalinen im XTT-Proliferationstest an nicht-resistenten und resistenten Tumorzelllinien.**

| | Proliferationsassay, EC₅₀ in [µM] | | | | | | | |
|---|---|---|---|---|---|---|---|---|
| **Vbb** | **A2780** | **A2780 cis-Pt** | **L1210** | **L1210 VCR** | **MESSA** | **MESSA DX5** | **NCI H69** | **NCI H69 AR** |
| 17 | 0.0003 | 0.0005 | 0.002 | 0.004 | 0.152 | 0.139 | 0.027 | 0.086 |
| 22 | 0.018 | 0.014 | 0.015 | 0.019 | 0.141 | 0.038 | 0.010 | 0.008 |
| 23 | 0.022 | 0.019 | 0.005 | 0.004 | 0.048 | 0.016 | 0.004 | 0.0008 |

Die erfinderischen Chinoxaline der Formel I zeigen eine sehr potente Hemmwirkung an allen getesteten Zelllinien.

## Patentansprüche

1. Chinoxalin-Derivate gemäß der allgemeinen Formel **I** 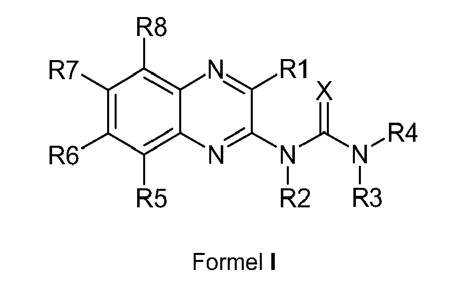 worin
X: Sauerstoff oder Schwefel;
R₁:
(i) Wasserstoff,
(ii) unsubstituiertes oder substituiertes (C₁-C₁₂)-Alkyl,
(iii) Cyano,
(iv) Halogen,
R₂ /R₃:
(i) Wasserstoff,
(ii) unsubstituiertes oder substituiertes (C₁-C₁₂)-Alkyl,
R₄:
(i) Wasserstoff,
(ii) unsubstituiertes oder substituiertes (C₁-C₁₂)-Alkyl,
(iii) unsubstituiertes oder substituiertes Cycloalkyl,
(iv) unsubstituiertes oder substituiertes Heterocyclyl,
(v) unsubstituiertes oder substituiertes Aryl,
(vi) unsubstituiertes oder substituiertes Heteroaryl,
(vii) unsubstituiertes oder substituiertes Alkyl-Aryl,
(viii) unsubstituiertes oder substituiertes Alkyl-Heteroaryl,
und
R₅-R₈:
(i) Wasserstoff,
(ii) unsubstituiertes oder substituiertes (C₁-C₁₂)-Alkyl,
(iii) unsubstituiertes oder substituiertes Aryl,
(iv) unsubstituiertes oder substituiertes Heteroaryl,
(v) Halogen,
(vi) Cyan,
(vii) Hydroxy,
(viii) (C₁-C₁₂)-Alkoxy,
(ix) Amino,
(x) Carboxyl, Alkoxycarbonyl, Carboxy-Alkyl oder Alkoxycarbonyl-Alkyl,
(xi) Alkoxycarbonylamino, Alkoxycarbonylamino-Alkyl bedeuten, und
mit der Maßgabe, dass mindestens einer der Substituenten R₅-R₈ einen unsubstituierten oder substituierten Aryl- oder Heteroaryl-Rest bedeutet,
und wobei die Substituenten ausgewählt sind aus der Gruppe bestehend aus F, Cl, Br, I, CN, CF₃, NH₂, NH-Alkyl, NH-Aryl, N(Alkyl)₂, NH-CO-Alkyl, NH-CO-Aryl, NH-CO-Heteroaryl, NH-SO₂-Alkyl, NH-SO₂-Aryl, NH-SO₂-Heteroaryl, NH-CO-NH-Alkyl, NH-CO-NH-Aryl, NH-CO-NH-Heteroaryl, NH-C(O)O-Alkyl, NH-C(O)O-Aryl, NH-C(O)O-Heteroaryl, NO₂, SH, S-Alkyl, OH, OCF₃, O-Alkyl, O-Aryl,O-CO-Alkyl, O-CO-Aryl, O-CO-Heteroaryl, O-C(O)O-Alkyl, O-C(O)O-Aryl, O-C(O)O-Heteroaryl, O-CO-NH-Alkyl, O-CO-N(Alkyl)₂, O-CO-NH-Aryl, O-CO-NH-Heteroaryl, OSO₃H, OSO₂-Alkyl, OSO₂-Aryl, OSO₂-Heteroaryl, OP(O)(OH)₂, Alkyl-P(O)(OH)₂ CHO, CO₂H, C(O)O-Alkyl, C(O)O-Aryl, C(O)O-Heteroaryl, CO-Alkyl, CO-Aryl, CO-Heteroaryl, SO₃H, SO₂-NH₂, SO₂-NH-Alkyl, SO₂-NH-Aryl, SO₂-NH-Heteroaryl, Alkyl, Cycloalkyl, Heterocyclyl, Aryl oder Heteroaryl, wobei die Reste "Alkyl", "Cycloalkyl", "Heterocyclyl", "Aryl" oder "Heteroaryl" ebenfalls substituiert sein können,
deren physiologisch verträgliche Salze, Hydrate, Solvate, wobei die Verbindungen der allgemeinen Formel (I) sowie deren Salze, Hydrate oder Solvate in Form ihrer Racemate, Enantiomeren und/oder Diastereomeren, oder in Form von Mischungen der Enantiomeren und/oder Diastereomeren, in Form der Tautomeren und deren polymorphen Formen vorliegen können.

2. Chinoxalin-Derivate der allgemeinen Formel **I** gemäß Anspruch 1, worin
Substituent X unabhängig Sauerstoff oder Schwefel;
Substituent R₁ unabhängig
(i) Wasserstoff,
(ii) unsubstituiertes oder substituiertes (C₁-C₁₂)-Alkyl, Substituenten R₂ /R₃ unabhängig
(i) Wasserstoff,
(ii) unsubstituiertes oder substituiertes (C₁-C₁₂)-Alkyl,
Substituent R₄ unabhängig
(i) Wasserstoff,
(ii) unsubstituiertes oder substituiertes (C₁-C₁₂)-Alkyl,
(iii) unsubstituiertes oder substituiertes (C₃-C₈)-Cycloalkyl,
(iv) unsubstituiertes oder substituiertes Piperidyl,
(v) unsubstituiertes oder substituiertes Phenyl, Naphthyl,
(vi) unsubstituiertes oder substituiertes Pyridyl, Isoxazolyl, Thiophenyl,
(vii) unsubstituiertes oder substituiertes Benzyl,
Substituenten R₅, R₇, R₈ unabhängig
(i) Wasserstoff,
(ii) unsubstituiertes oder substituiertes (C₁-C₁₂)-Alkyl,
und
Substituent R₆
(i) unsubstituiertes oder substituiertes Phenyl, bedeuten
und wobei die Substituenten ausgewählt sind aus der Gruppe bestehend aus F, Cl, Br, I, CN, CF₃, NH₂, NH-Alkyl, NH-Aryl, N(Alkyl)₂, NH-CO-Alkyl, NH-CO-Aryl, NH-CO-Heteroaryl, NH-SO₂-Alkyl, NH-SO₂-Aryl, NH-SO₂-Heteroaryl, NH-CO-NH-Alkyl, NH-CO-NH-Aryl, NH-CO-NH-Heteroaryl, NH-C(O)O-Alkyl, NH-C(O)O-Aryl, NH-C(O)O-Heteroaryl, NO₂, SH, S-Alkyl, OH, OCF₃, O-Alkyl, O-Aryl,O-CO-Alkyl, O-CO-Aryl, O-CO-Heteroaryl, O-C(O)O-Alkyl, O-C(O)O-Aryl, O-C(O)O-Heteroaryl, O-CO-NH-Alkyl, O-CO-N(Alkyl)₂, O-CO-NH-Aryl, O-CO-NH-Heteroaryl, OSO₃H, OSO₂-Alkyl, OSO₂-Aryl, OSO₂-Heteroaryl, OP(O)(OH)₂, Alkyl-P(O)(OH)₂ CHO, CO₂H, C(O)O-Alkyl, C(O)O-Aryl, C(O)O-Heteroaryl, CO-Alkyl, CO-Aryl, CO-Heteroaryl, SO₃H, SO₂-NH₂, SO₂-NH-Alkyl, SO₂-NH-Aryl, SO₂-NH-Heteroaryl, Alkyl, Cycloalkyl, Heterocyclyl, Aryl oder Heteroaryl,
wobei die Reste "Alkyl", "Cycloalkyl", "Heterocyclyl", "Aryl" oder "Heteroaryl" ebenfalls substituiert sein können

3. Chinoxalin-Derivate der allgemeinen Formel **I** gemäß Anspruch 1, worin
Substituent X unabhängig Sauerstoff oder Schwefel;
R₁ unabhängig
(i) Wasserstoff,
(ii) unsubstituiertes oder substituiertes (C₁-C₁₂)-Alkyl,
(iii) Cyano,
(iv) Halogen,
R₂ /R₃ unbhängig
(i) Wasserstoff,
(ii) unsubstituiertes oder substituiertes (C₁-C₁₂)-Alkyl,
R₄ unabhängig
(i) Wasserstoff,
(ii) unsubstituiertes oder substituiertes (C₁-C₁₂)-Alkyl,
(iii) unsubstituiertes oder substituiertes Cycloalkyl,
(iv) unsubstituiertes oder substituiertes Heterocyclyl,
(v) unsubstituiertes oder substituiertes Aryl,
(vi) unsubstituiertes oder substituiertes Heteroaryl,
(vii) unsubstituiertes oder substituiertes Alkyl-Aryl,
(viii) unsubstituiertes oder substituiertes Alkyl-Heteroaryl,
R₅, R₇ ,R₈ unabhängig
(i) Wasserstoff,
(ii) unsubstituiertes oder substituiertes (C₁-C₁₂)-Alkyl,
(iii) unsubstituiertes oder substituiertes Aryl,
(iv) unsubstituiertes oder substituiertes Heteroaryl,
(v) Halogen,
(vi) Cyan,
(vii) Hydroxy,
(viii) (C₁-C₁₂)-Alkoxy,
(ix) Amino,
(x) Carboxyl, Alkoxycarbonyl, Carboxy-Alkyl oder Alkoxycarbonyl-Alkyl,
(xi) Alkoxycarbonylamino, Alkoxycarbonylamino-Alkyl bedeuten,
wobei die Substituenten ausgewählt sind aus der Gruppe bestehend aus F, Cl, Br, I, CN, CF₃, NH₂, NH-Alkyl, NH-Aryl, N(Alkyl)₂, NH-CO-Alkyl, NH-CO-Aryl, NH-CO-Heteroaryl, NH-SO₂-Alkyl, NH-SO₂-Aryl, NH-SO₂-Heteroaryl, NH-CO-NH-Alkyl, NH-CO-NH-Aryl, NH-CO-NH-Heteroaryl, NH-C(O)O-Alkyl, NH-C(O)O-Aryl, NH-C(O)O-Heteroaryl, NO₂, SH, S-Alkyl, OH, OCF₃, O-Alkyl, O-Aryl,O-CO-Alkyl, O-CO-Aryl, O-CO-Heteroaryl, O-C(O)O-Alkyl, O-C(O)O-Aryl, O-C(O)O-Heteroaryl, O-CO-NH-Alkyl, O-CO-N(Alkyl)₂, O-CO-NH-Aryl, O-CO-NH-Heteroaryl, OSO₃H, OSO₂-Alkyl, OSO₂-Aryl, OSO₂-Heteroaryl, OP(O)(OH)₂, Alkyl-P(O)(OH)₂ CHO, CO₂H, C(O)O-Alkyl, C(O)O-Aryl, C(O)O-Heteroaryl, CO-Alkyl, CO-Aryl, CO-Heteroaryl, SO₃H, SO₂-NH₂, SO₂-NH-Alkyl, SO₂-NH-Aryl, SO₂-NH-Heteroaryl, Alkyl, Cycloalkyl, Heterocyclyl, Aryl oder Heteroaryl,
wobei die Reste "Alkyl", "Cycloalkyl", "Heterocyclyl", "Aryl" oder "Heteroaryl" ebenfalls substituiert sein können,
und
R₆ unabhängig
(i) unsubstituiertes oder substituiertes Aryl,
(ii) unsubstituiertes oder substituiertes Heteroaryl bedeuten,
wobei die Substituenten ausgewählt sind aus der Gruppe, bestehend aus Halogen, (C₁-C₁₂)-Alkyl, Hydroxy, (C₁-C₁₂)-Alkoxy.

4. Chinoxalin-Derivate der allgemeinen Formel **I** gemäß einem der Ansprüche 1 bis 3, ausgewählt aus der Gruppe bestehend aus:
Cyclopentyl-3-[7-(4-hydroxy-3-methoxy-phenyl)-quinoxalin-2-yl]-urea **(Verbindung 1)** 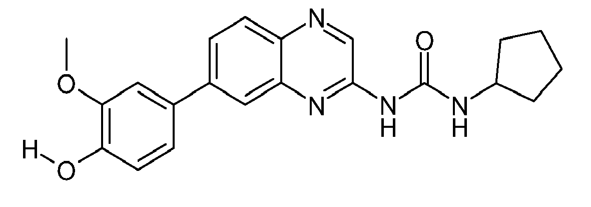
1-Cyclohexyl-3-[7-(4-hydroxy-3-methoxy-phenyl)-quinoxalin-2-yl]-urea **(Verbindung 2)** 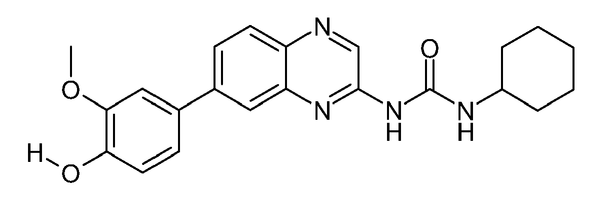
Cyclohexyl-3-[7-(3,4-dimethoxy-phenyl)-quinoxalin-2-yl]-urea **(Verbindung 3)** 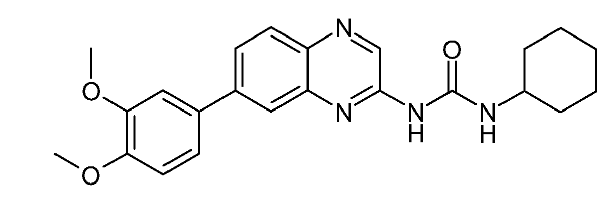
1-[7-(3,4-Dimethoxy-phenyl)-quinoxalin-2-yl]-3-[1-(2,2,2-trifluoro-acetyl)-piperidin-4-yl]-urea **(Verbindung 4)** 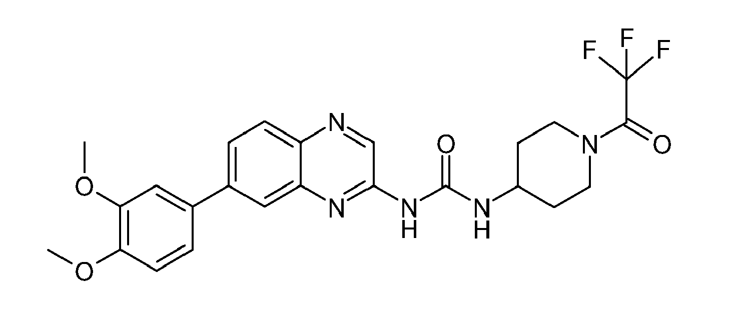
1-[7-(4-Hydroxy-3-methoxy-phenyl)-quinoxalin-2-yl]-3-phenyl-urea **(Verbindung 5)** 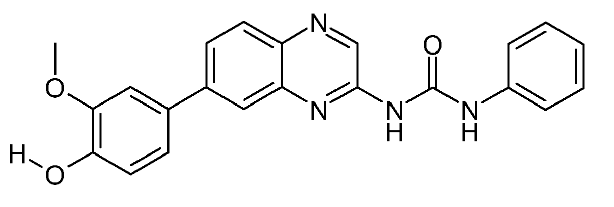
1-[7-(4-Hydroxy-3-methoxy-phenyl)-quinoxalin-2-yl]-3-[1-(2,2,2-trifluoro-acetyl)-piperidin-4-yl]-urea **(Verbindung 6)** 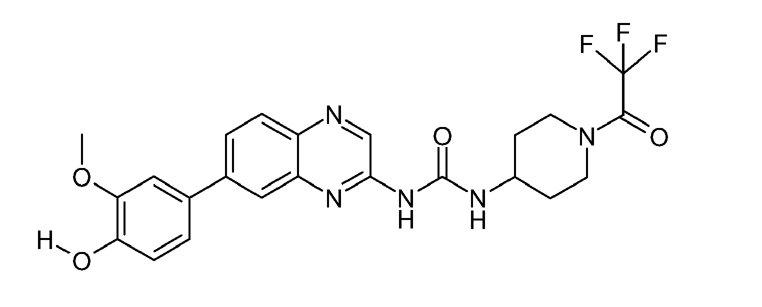
1-Benzyl-3-[7-(4-hydroxy-3-methoxy-phenyl)-quinoxalin-2-yl]-urea **(Verbindung 7)** 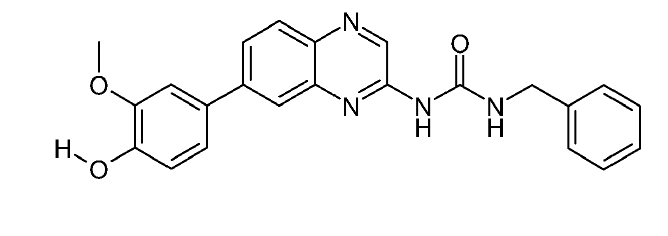
1-Cyclopentyl-3-[7-(3,4-dimethoxy-phenyl)-quinoxalin-2-yl]-urea **(Verbindung 8)** 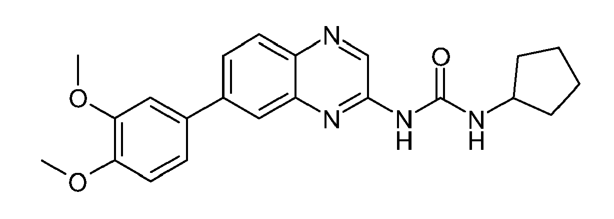
1-tert-Butyl-3-[7-(4-hydroxy-3,5-dimethyl-phenyl)-quinoxalin-2-yl]-urea **(Verbindung 9)** 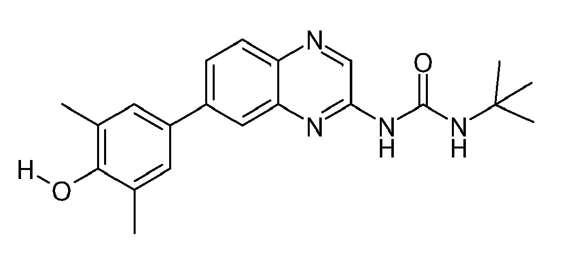
1-tert-Butyl-3-[7-(4-hydroxy-3-methoxy-phenyl)-quinoxalin-2-yl]-urea **(Verbindung 10)** 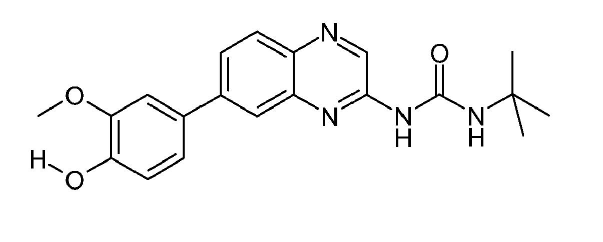
1-[7-(4-Hydroxy-3-methoxy-phenyl)-quinoxalin-2-yl]-3-thiophen-2-yl-urea **(Verbindung 11)** 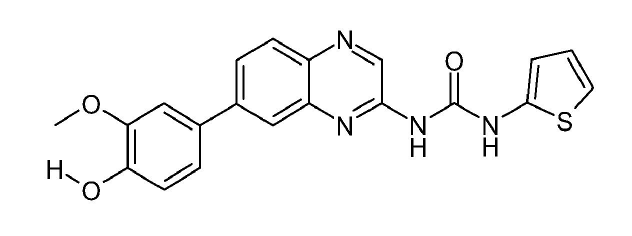
1-Cyclohexyl-3-[7-(4-hydroxy-3,5-dimethyl-phenyl)-quinoxalin-2-yl]-urea **(Verbindung 12)** 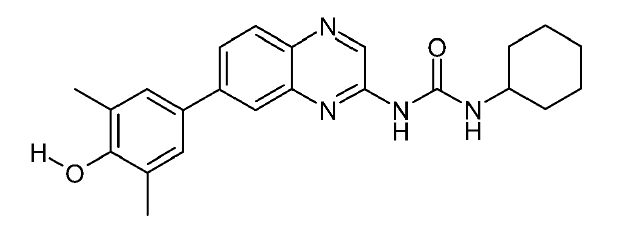
1-Cyclopentyl-3-[7-(4-hydroxy-3,5-dimethyl-phenyl)-quinoxalin-2-yl]-urea **(Verbindung 13)** 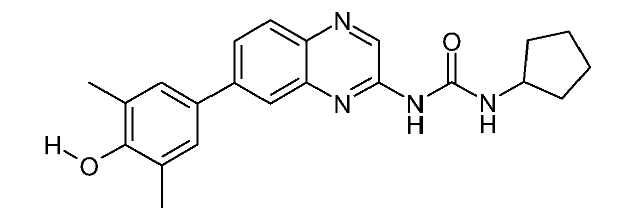
1-[7-(4-Hydroxy-3,5-dimethyl-phenyl)-quinoxalin-2-yl]-3-phenyl-urea **(Verbindung 14)** 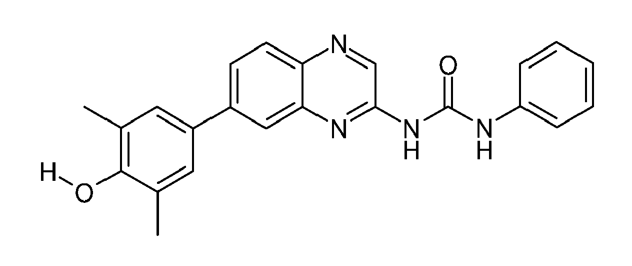
1-[7-(4-Hydroxy-3,5-dimethyl-phenyl)-quinoxalin-2-yl]-3-thiophen-2-yl-urea **(Verbindung 15)** 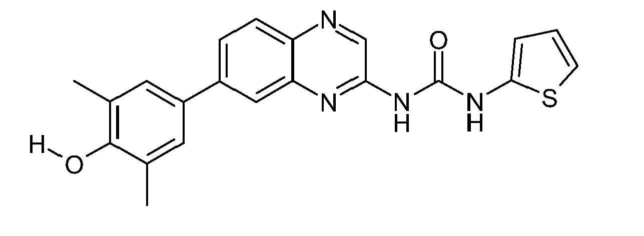
1-[7-(4-Hydroxy-3,5-dimethyl-phenyl)-quinoxalin-2-yl]-3-methyl-urea **(Verbindung 16)** 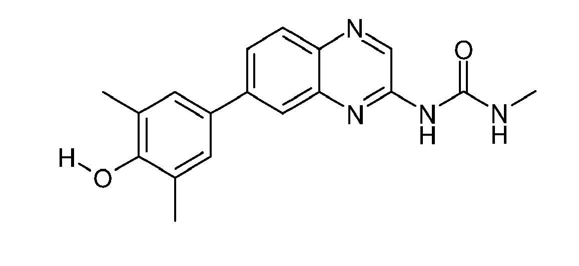 1-[7-(4-Hydroxy-3,5-dimethyl-phenyl)-quinoxalin-2-yl]-3-isopropyl-urea **(Verbindung 17)** 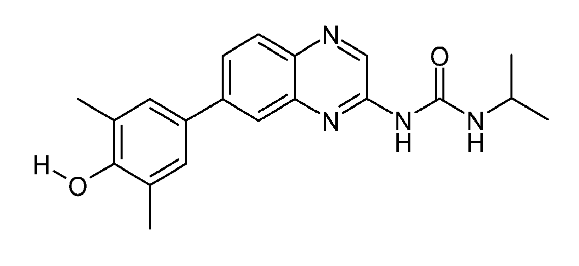
1-Butyl-3-[7-(4-hydroxy-3,5-dimethyl-phenyl)-quinoxalin-2-yl]-urea **(Verbindung 18)** 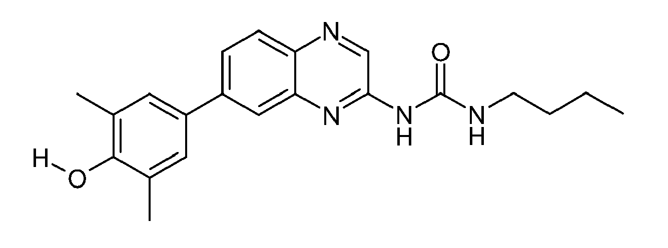 1-Ethyl-3-[7-(4-hydroxy-3,5-dimethyl-phenyl)-quinoxalin-2-yl]-urea **(Verbindung 19)** 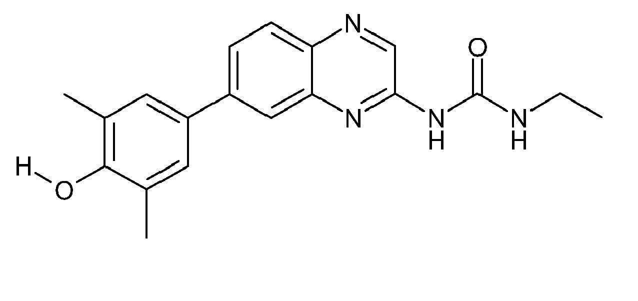
1-Cyclohexyl-3-[7-(4-hydroxy-3,5-dimethyl-phenyl)-quinoxalin-2-yl]-thiourea **(Verbindung 20)** 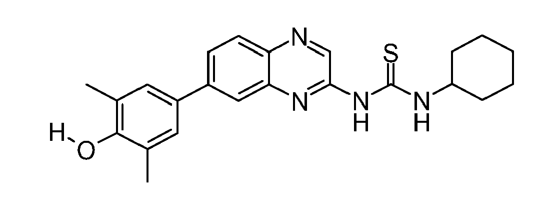
1-Cyclohexyl-3-[7-(3,5-dichloro-4-hydroxy-phenyl)-quinoxalin-2-yl]-thiourea **(Verbindung 21)** 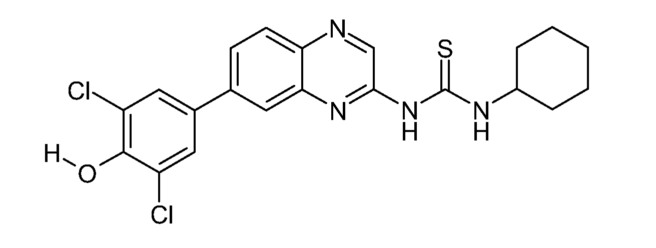
1-Ethyl-3-[7-(4-hydroxy-3,5-dimethyl-phenyl)-quinoxalin-2-yl]-thiourea **(Verbindung 22)** 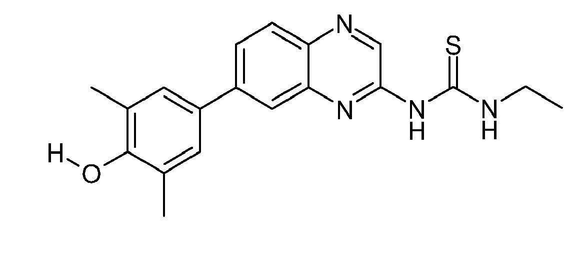
1-Cyclopropyl-3-[7-(4-hydroxy-3,5-dimethyl-phenyl)-quinoxalin-2-yl]-thiourea **(Verbindung 23)** 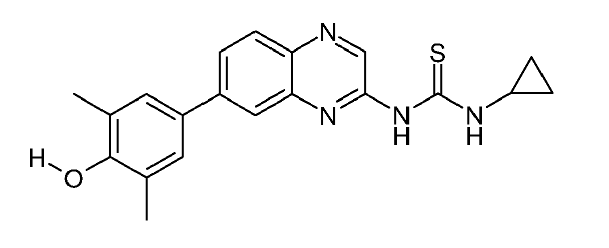
1-Cyclopentyl-3-[7-(4-hydroxy-3,5-dimethyl-phenyl)-quinoxalin-2-yl]-thiourea **(Verbindung 24)** 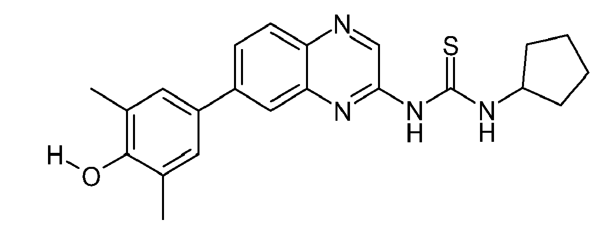
1-tert-Butyl-3-[7-(4-hydroxy-3,5-dimethyl-phenyl)-quinoxalin-2-yl]-thiourea **(Verbindung 25)** 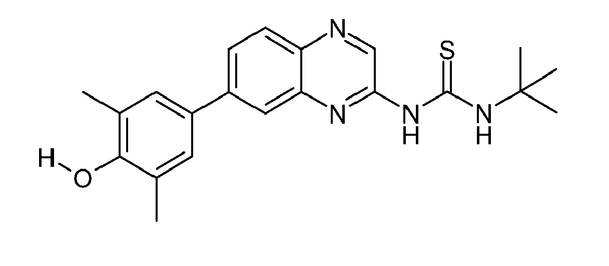
1-Dodecyl-3-(7-phenyl-quinoxalin-2-yl)-urea **(Verbindung 26)** 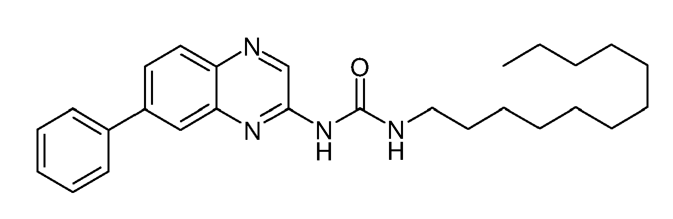
1-(3-Chloro-2-methyl-phenyl)-3-(7-phenyl-quinoxalin-2-yl)-urea **(Verbindung 27)** 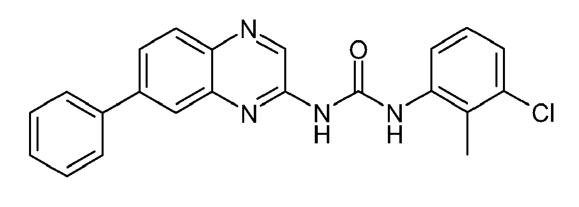
1-(3,4-Dimethyl-phenyl)-3-(7-phenyl-quinoxalin-2-yl)-urea **(Verbindung 28)** 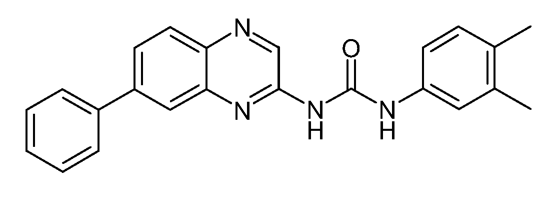
1-Allyl-3-(7-phenyl-quinoxalin-2-yl)-urea **(Verbindung 29)** 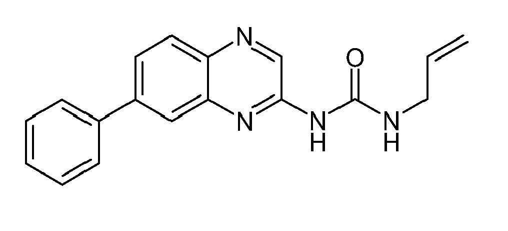
1-Cyclopentyl-3-(7-phenyl-quinoxalin-2-yl)-urea **(Verbindung 30)** 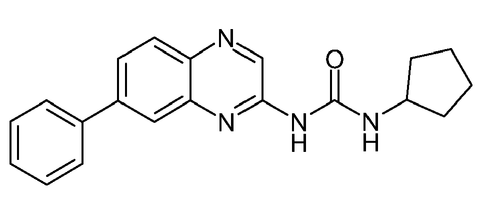
1-[7-(4-Hydroxy-3,5-dimethyl-phenyl)-quinoxalin-2-yl]-3-isopropyl-thiourea **(Verbindung 31)** 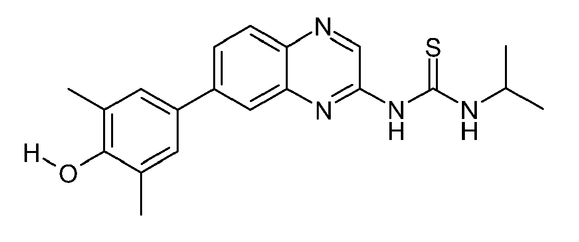
1-Butyl-3-[7-(4-hydroxy-3,5-dimethyl-phenyl)-quinoxalin-2-yl]-thiourea **(Verbindung 32)** 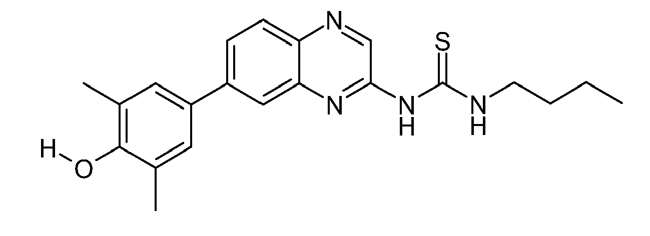
1-[7-(4-Hydroxy-3,5-dimethyl-phenyl)-quinoxalin-2-yl]-3-pentyl-urea **(Verbindung 33)** 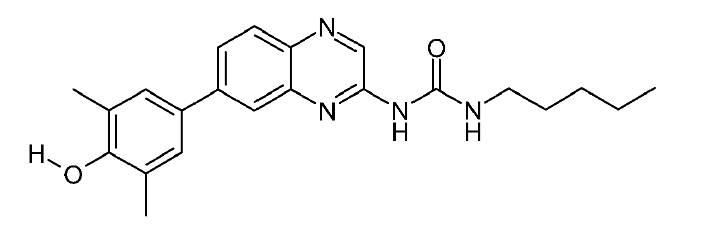
1-Cyclobutyl-3-[7-(4-hydroxy-3,5-dimethyl-phenyl)-quinoxalin-2-yl]-thiourea **(Verbindung 34)** 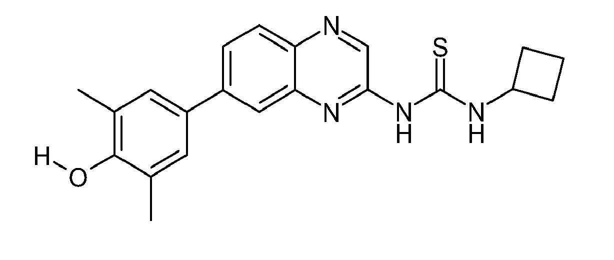
1-Hexyl-3-[7-(4-hydroxy-3,5-dimethyl-phenyl)-quinoxalin-2-yl]-urea **(Verbindung 35)** 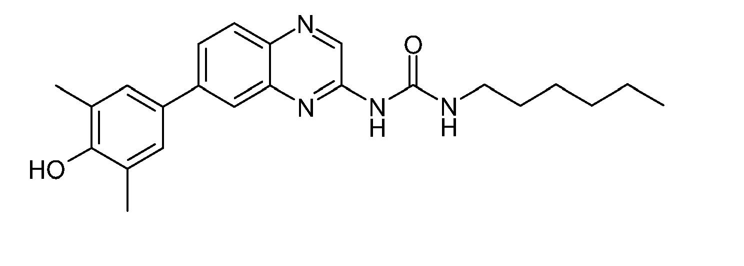
1-[7-(4-Hydroxy-3,5-dimethyl-phenyl)-quinoxalin-2-yl]-3-propyl-urea **(Verbindung 36)** 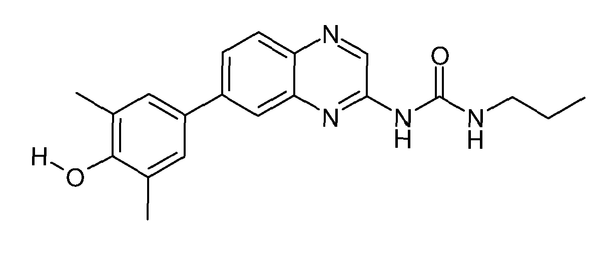
1-Dodecyl-3-[7-(4-hydroxy-3,5-dimethyl-phenyl)-quinoxalin-2-yl]-urea **(Verbindung 37)** 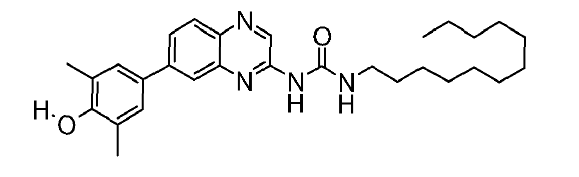
1-(3-Chloro-2-methyl-phenyl)-3-[7-(4-hydroxy-3,5-dimethyl-phenyl)-quinoxalin-2-yl]-urea **(Verbindung 38)** 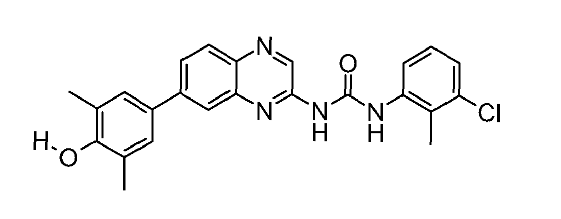
1-(3-Acetyl-phenyl)-3-[7-(4-hydroxy-3,5-dimethyl-phenyl)-quinoxalin-2-yl]-urea **(Verbindung 39)** 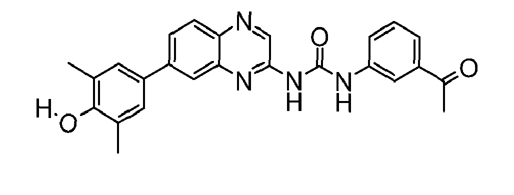
1-(3,4-Dimethyl-phenyl)-3-[7-(4-hydroxy-3,5-dimethyl-phenyl)-quinoxalin-2-yl]-urea **(Verbindung 40)** 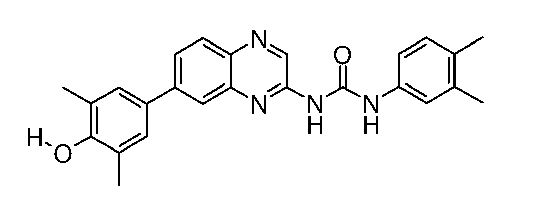
1-Allyl-3-[7-(4-hydroxy-3,5-dimethyl-phenyl)-quinoxalin-2-yl]-urea **(Verbindung 41)** 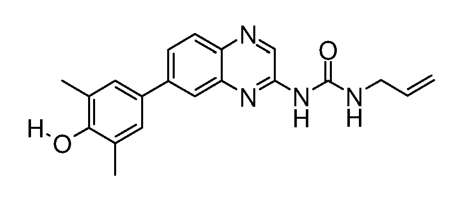
1-Cyclooctyl-3-[7-(4-hydroxy-3,5-dimethyl-phenyl)-quinoxalin-2-yl]-urea **(Verbindung 42)** 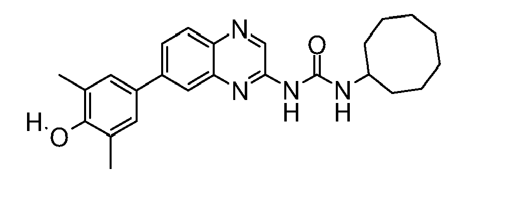
1-(2,4-Dimethyl-phenyl)-3-[7-(4-hydroxy-3,5-dimethyl-phenyl)-quinoxalin-2-yl]-urea **(Verbindung 43)** 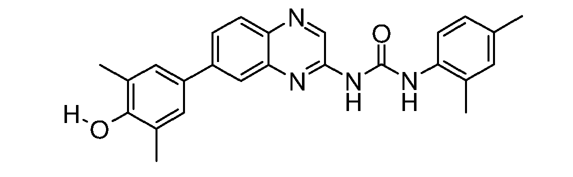
1-Cyclooctyl-3-[7-(4-hydroxy-3-methoxy-phenyl)-quinoxalin-2-yl]-urea **(Verbindung 44)** 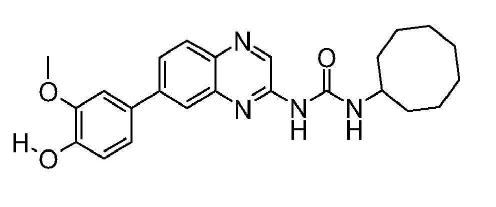
1-Adamantan-1-yl-3-(7-phenyl-quinoxalin-2-yl)-urea **(Verbindung 45)** 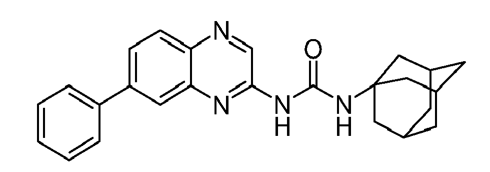
1-[7-(4-Hydroxy-3,5-dimethyl-phenyl)-quinoxalin-2-yl]-3-p-tolyl-urea **(Verbindung 46)** 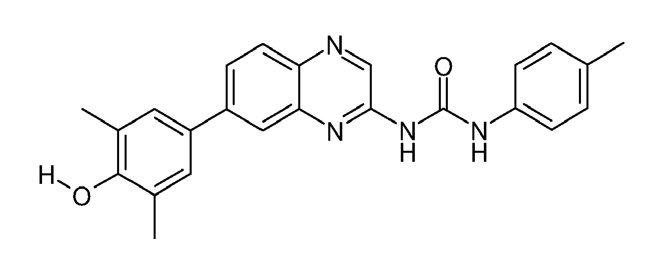
1-(3,4-Dichloro-phenyl)-3-[7-(4-hydroxy-3,5-dimethyl-phenyl)-quinoxalin-2-yl]-urea **(Verbindung 47)** 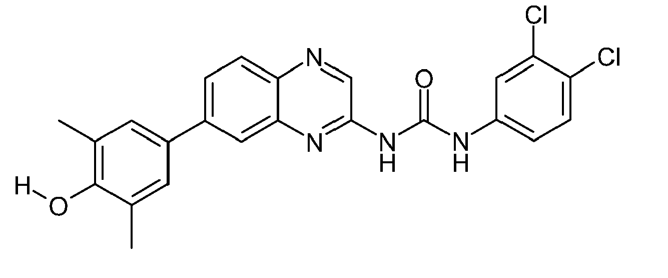
1-[7-(4-Hydroxy-3,5-dimethyl-phenyl)-quinoxalin-2-yl]-3-(4-methoxy-phenyl)-urea **(Verbindung 48)** 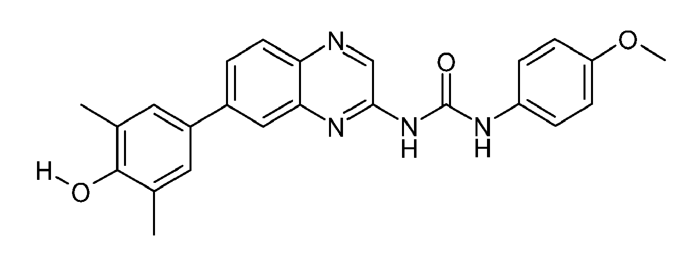
1-Adamantan-1-yl-3-[7-(4-hydroxy-3,5-dimethyl-phenyl)-quinoxalin-2-yl]-urea **(Verbindung 49)** 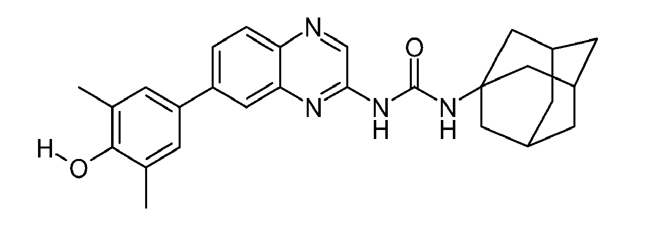
1-[7-(4-Hydroxy-3,5-dimethyl-phenyl)-quinoxalin-2-yl]-3-naphthalen-2-yl-urea **(Verbindung 50)** 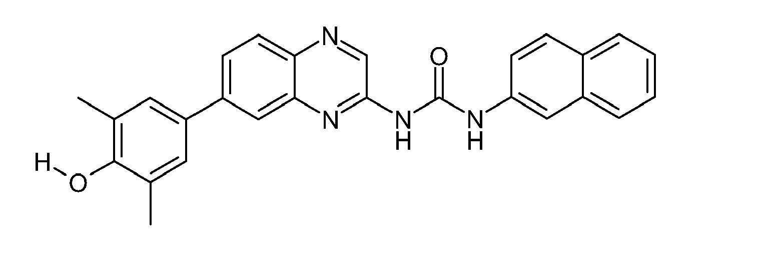
1-[7-(4-Hydroxy-3,5-dimethyl-phenyl)-quinoxalin-2-yl]-3-(1,1,3,3-tetramethyl-butyl)-urea **(Verbindung 51)** 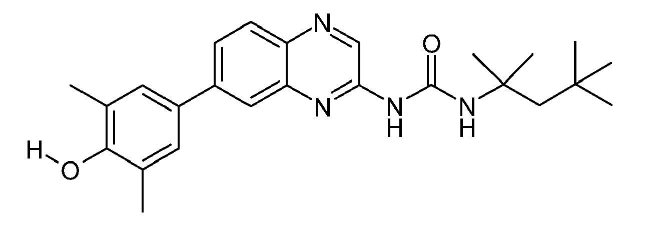
1-[7-(4-Hydroxy-3,5-dimethyl-phenyl)-quinoxalin-2-yl]-3-pyridin-3-yl-urea **(Verbindung 52)** 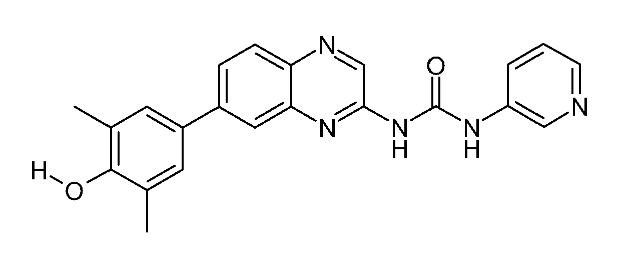
1-((R)-1,2-Dimethyl-propyl)-3-[7-(4-hydroxy-3,5-dimethyl-phenyl)-quinoxalin-2-yl]-urea **(Verbindung 53)** 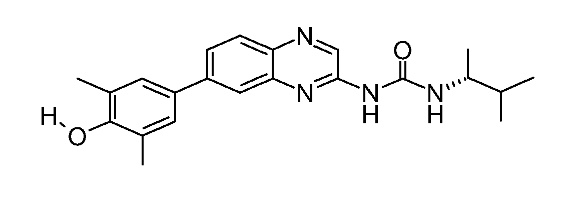
1-((S)-1,2-Dimethyl-propyl)-3-[7-(4-hydroxy-3,5-dimethyl-phenyl)-quinoxalin-2-yl]-urea **(Verbindung 54)** 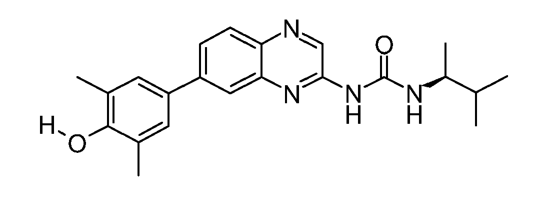
1-(5-Chloro-2-methyl-phenyl)-3-[7-(4-hydroxy-3,5-dimethyl-phenyl)-quinoxalin-2-yl]-urea **(Verbindung 55)** 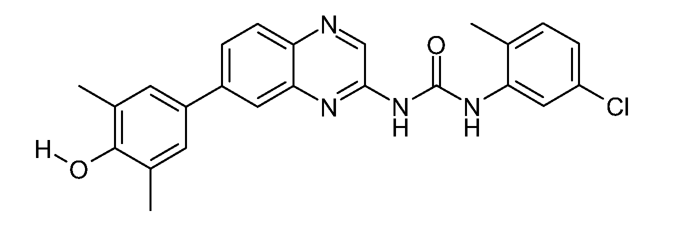
1-((S)-2-Phenyl-cyclopropyl)-3-(7-phenyl-quinoxalin-2-yl)-urea **(Verbindung 56)** 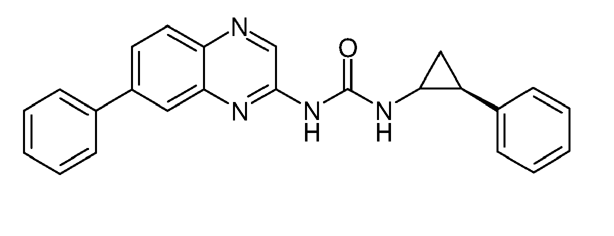
1-[7-(4-Hydroxy-3,5-dimethyl-phenyl)-quinoxalin-2-yl]-3-((S)-2-phenyl-cyclopropyl)-urea **(Verbindung 57)** 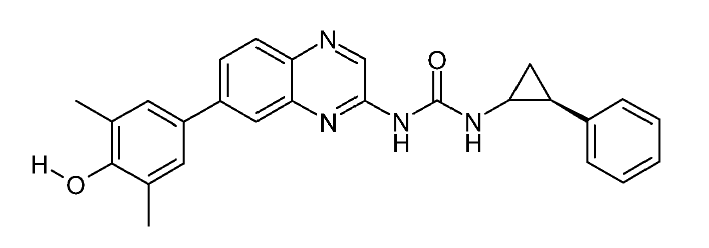
1-(2-Chloro-6-methyl-phenyl)-3-[7-(4-hydroxy-3,5-dimethyl-phenyl)-quinoxalin-2-yl]-urea **(Verbindung 58)** 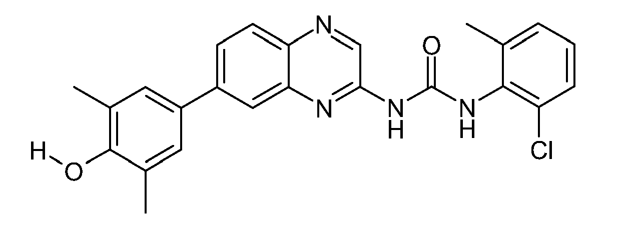
1-Allyl-3-[7-(4-hydroxy-3-methoxy-phenyl)-quinoxalin-2-yl]-urea **(Verbindung 59)** 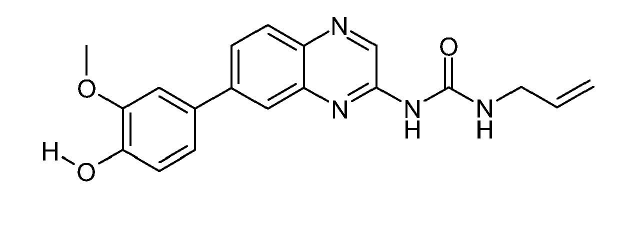
1-(3,5-Dimethyl-isoxazol-4-yl)-3-[7-(4-hydroxy-3,5-dimethyl-phenyl)-quinoxalin-2-yl]-urea **(Verbindung 60)** 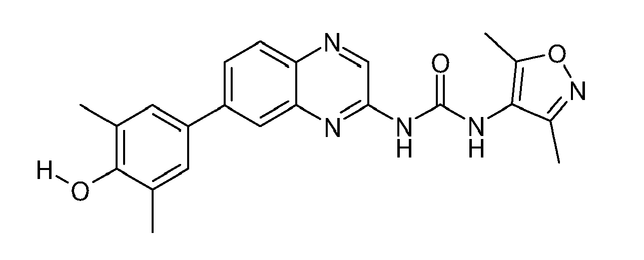
1-sec-Butyl-3-[7-(4-hydroxy-3,5-dimethyl-phenyl)-quinoxalin-2-yl]-urea **(Verbindung 61)** 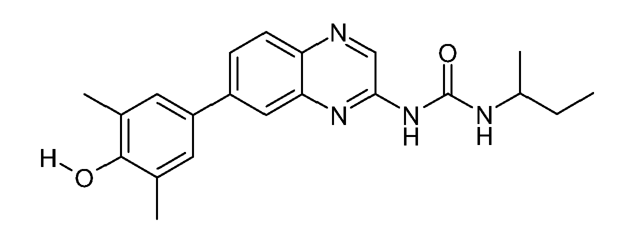
1-Ethyl-3-[7-(4-methoxy-3,5-dimethyl-phenyl)-quinoxalin-2-yl]-urea **(Verbindung 62)** 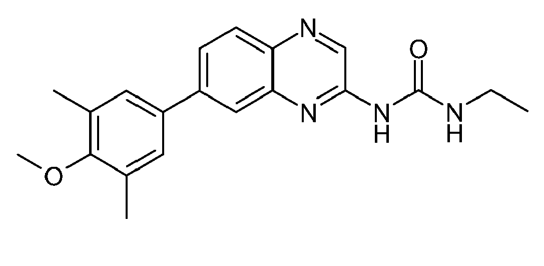
1-Isopropyl-3-[7-(4-methoxy-3,5-dimethyl-phenyl)-quinoxalin-2-yl]-urea **(Verbindung 63)** 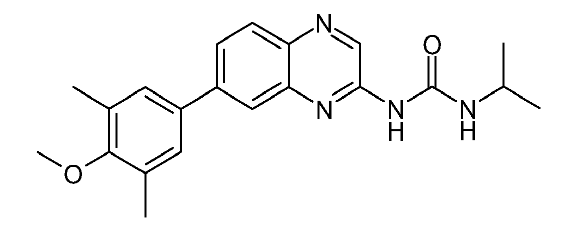
1-Isopropyl-3-[7-(4-methoxy-3,5-dimethyl-phenyl)-quinoxalin-2-yl]-thiourea **(Verbindung 64)** 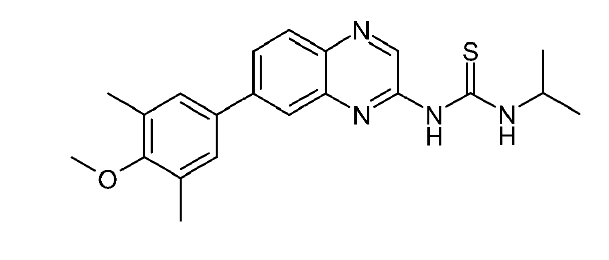
1-[7-(4-Hydroxy-3,5-dimethyl-phenyl)-quinoxalin-2-yl]-3-isobutyl-urea **(Verbindung 65)** 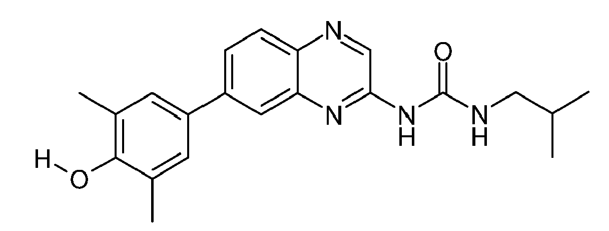
1-(-Ethyl-propyl)-3-[7-(4-hydroxy-3,5-dimethyl-phenyl)-quinoxalin-2-yl]-urea **(Verbindung 66)**

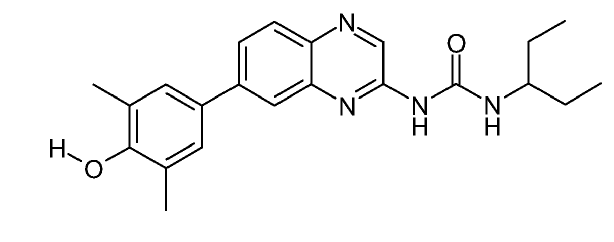

5. Pharmazeutische Zusammensetzung, enthaltend eine pharmakologisch aktive Menge mindestens einer Verbindung gemäß einem der Ansprüche 1 bis 4.

6. Pharmazeutische Zusammensetzung gemäß Anspruch 5, wobei die Zusammensetzung mindestens eine weitere pharmakologisch aktive Substanz enthält.

7. Pharmazeutische Zusammensetzung gemäß einem der Ansprüche 5 oder 6, wobei die Zusammensetzung zusätzlich pharmazeutisch akzeptable Träger- und/oder Hilfsstoffe enthält.

8. Pharmazeutische Zusammensetzung gemäß einem der Ansprüche 5-7, wobei die weitere pharmakologisch aktive Substanz ausgewählt ist aus der Gruppe bestehend aus:
Asparaginase, Bleomycin, Carboplatin, Carmustine, Chlorambucil, Cisplatin, Colaspase, Cyclophosphamide, Cytarabine, Dacarbazine, Dactinomycin, Daunorubicin, Doxorubicin (Adriamycin), Epirubicin, Etoposide, 5-Fluorouracil, Hexamethylmelamine, Hydroxyurea, Ifosfamide, Irinotecan, Leucovorin, Lomustine, Mechlorethamine, 6-Mercaptopurine, Mesna, Methotrexate, Mitomycin C, Mitoxantrone, Prednisolone, Prednisone, Procarbazine, Raloxifen, Streptozocin, Tamoxifen, Thalidomide, Thioguanine, Topotecan, Vinblastine, Vincristine, Vindesine, Aminoglutethimide, L-Asparaginase, Azathioprine, 5-Azacytidine cladribine, Busulfan, Diethylstilbestrol, 2',2'-Difluorodeoxycytidine, Docetaxel, Erythrohydroxynonyladenine, Ethynylestradiol, 5-Fluorodeoxyuridine, 5-Fluorodeoxyuridine monophosphate, Fludarabine phosphate, Fluoxymesterone, Flutamide, Hydroxyprogesterone caproate, Idarubicin, Interferon, Medroxyprogesterone acetate, Megestrol acetate, Melphalan, Mitotane, Paclitaxel, Oxaliplatin, Pentostatin, N-Phosphonoacetyl-L-aspartate (PALA), Plicamycin, Semustine, Teniposide, Testosterone propionate, Thiotepa, Trimethylmelamine, Uridine, Vinorelbine, Epothilone, Gemcitabine, Taxotere, BCNU, CCNU, DTIC, Herceptin, Avastin, Erbitux, Sorafenib, Gleevec, Iressa, Tarceva, Rapamycin, Actinomycin D, Sunitinib (sutent).

9. Verbindung gemäß einem der Ansprüche 1 bis 4 zur Verwendung als Arzneimittel.

10. Verwendung einer Verbindung gemäß einem der Ansprüche 1 bis 4 zur Herstellung eines Arzneimittels zur Behandlung von Tumorerkrankungen.

11. Verwendung einer Verbindung gemäß einem der Ansprüche 1 bis 4 und 10 zur Herstellung eines Arzneimittels zur Behandlung von wirkstoffresistenten Tumorerkrankungen.

12. Verwendung einer Verbindung gemäß einem der Ansprüche 1 bis 4 und 10 zur Herstellung eines Arzneimittels zur Behandlung von metastasierenden Karzinomen.

13. Verwendung gemäß einem der Ansprüche 10 bis 12, wobei das Arzneimittel zusätzlich mindestens eine weitere pharmakologisch aktive Substanz enthält.

14. Verwendung gemäß Anspruch 13, wobei die weitere pharmakologisch aktive Substanz ausgewählt ist aus der Gruppe bestehend aus:
Asparaginase, Bleomycin, Carboplatin, Carmustine, Chlorambucil, Cisplatin, Colaspase, Cyclophosphamide, Cytarabine, Dacarbazine, Dactinomycin, Daunorubicin,
Doxorubicin (Adriamycin), Epirubicin, Etoposide, 5-Fluorouracil, Hexamethylmelamine, Hydroxyurea, Ifosfamide, Irinotecan, Leucovorin, Lomustine, Mechlorethamine, 6-Mercaptopurine, Mesna, Methotrexate, Mitomycin C, Mitoxantrone, Prednisolone, Prednisone, Procarbazine, Raloxifen, Streptozocin, Tamoxifen, Thalidomide, Thioguanine, Topotecan, Vinblastine, Vincristine, Vindesine, Aminoglutethimide, L-Asparaginase, Azathioprine, 5-Azacytidine cladribine, Busulfan, Diethylstilbestrol, 2',2'-Difluorodeoxycytidine, Docetaxel, Erythrohydroxynonyladenine, Ethynylestradiol, 5-Fluorodeoxyuridine, 5-Fluorodeoxyuridine monophosphate, Fludarabine phosphate, Fluoxymesterone, Flutamide, Hydroxyprogesterone caproate, Idarubicin, Interferon, Medroxyprogesterone acetate, Megestrol acetate, Melphalan, Mitotane, Paclitaxel, Oxaliplatin, Pentostatin, N-Phosphonoacetyl-L-aspartate (PALA), Plicamycin, Semustine, Teniposide, Testosterone propionate, Thiotepa, Trimethylmelamine, Uridine, Vinorelbine, Epothilone, Gemcitabine, Taxotere, BCNU, CCNU, DTIC, Herceptin, Avastin, Erbitux, Sorafenib, Gleevec, Iressa, Tarceva, Rapamycin, Actinomycin D, Sunitinib (sutent).

15. Arzneimittel zur Behandlung von Tumorerkrankungen, enthaltend mindestens ein Chinoxalin-Derivat gemäß einem der Ansprüche 1 bis 4.

16. Arzneimittel gemäß Anspruch 15, enthaltend ein Chinoxalin-Derivat in Kombination mit mindestens einem weiteren pharmazeutischen Wirkstoff und/oder pharmazeutisch akzeptablen Träger- und/oder Hilfsstoffen.

17. Verfahren zur Herstellung eines Arzneimittels nach einem der Ansprüche 15 bis 16, **dadurch gekennzeichnet, dass** ein oder mehrere Chinoxalin-Derivate gemäß einem der Ansprüche 1 bis 3 mit akzeptablen Träger- und/oder Hilfsstoffen zu pharmazeutischen Zubereitungen verarbeitet, beziehungsweise in eine therapeutisch anwendbare Form gebracht werden.
